# EUROPEAN PATENT APPLICATION

(11) **EP 1 600 503 A1**
(43) Date of publication of application: **30.11.2005**
(21) Application number: 04011592.5
(22) Date of filing: 14.05.2004
(51) Int. Cl.: C12N 9/04, C12Q 1/32, C12N 15/53, C12N 15/63, C12N 5/10

(54) **Modified pyrroloquinoline quinone-dependent glucose dehydrogenase**

(71) Applicant: Amano Enzyme Inc., Nagoya-shi, Aichi 460-0003 (JP)
(72) Inventor: Suzumura, Akitoshi c/o Gifu R&D Center, Kakamigahara-sgi, Gifu 509-0108 (JP); Hamamatsu, Norio c/o Tsukuba Research Institute, Tsukuba 300-2611 (JP)
(74) Representative: Müller Fottner Steinecke

(57) **Abstract**

A modified pyrroloquinoline quinone-dependent glucose dehydrogenase having a low reactivity with respect to maltose, galactose, etc. is provided. A modified pyrroloquinoline quinone-dependent glucose dehydrogenase including an amino acid sequence in which one or more amino acids in a region corresponding to a first region consisting of amino acids at positions 326 to 354 in pyrroloquinoline quinone-dependent glucose dehydrogenase derived from Acinetobacter calcoaceticus are substituted as compared with an amino acid sequence of the corresponding wild-type enzyme.

## Description

### TECHNICAL FIELD

The present invention relates to a pyrroloquinoline quinone-dependent glucose dehydrogenase. More particularly, the present invention relates to a modified pyrroloquinoline quinone-dependent glucose dehydrogenase in which an amino acid in a certain region is substituted by another amino acid as compared with a wild-type enzyme. The modified pyrroloquinoline quinone-dependent glucose dehydrogenase of the present invention is used for measurement of an amount of glucose in, for example, a clinical test.

### BACKGROUND ART

A pyrroloquinoline quinone-dependent β-D glucose dehydrogenase (EC1.1.199, PQQGDH) is conjugated with a coenzyme, pyrrolo-quinoline quinone (PQQ) to catalyze a reaction of oxidizing β-D glucose to produce glucono-lactone. With this property, PQQGDH is used for determining an amount of glucose in a clinical test, a food analysis, monitoring of a culture process, and the like.

As examples of PQQGDH reported in the past, a PQQGDH produced by Acinetobacter species L.M.D 79.41 strain and a modified PQQGDH thereof are known (for example, JP 2000-312588A, JP 2000-350588A, JP 2000-197888A, JP 2001-346587A, W002-34919 (A1), and A-M Cleton-Jansen et al., Mol. Gen. Genet., 217, 430 (1989)). The substrate specificity of the PQQGDH produced by L.M.D 79.41 strain is low. For example, also with respect to maltose, the reactivity of the PQQGDH has a reactivity corresponding to about 90% of the reactivity with respect to glucose.

### DISCLOSURE OF INVENTION

In order to measure an exact amount of glucose, a PQQGDH is required to have high substrate specificity. For example, when carrying out diagnosis of diabetes for patients who undergo drip transfusion of blood, if PQQGDH having low substrate specificity is used, the obtained measurement value includes the amount of maltose in a transfusion liquid in addition to the amount of glucose. Thus, the exact amount of glucose in blood cannot be determined. Similarly, for example, when carrying out measurement for patients with hepatic disorder, if PQQGDH having low substrate specificity is used, due to the influence of galactose, reliable measurement may not be carried out. In this way, since PQQGDH having low substrate specificity is much influenced by other sugars such as maltose, an exact amount of glucose cannot be measured.

In the above-mentioned modified PQQGDH reported in the past, by substituting a part of amino acids of the wild-type PQQGDH, the substrate specificity is improved. However, reactivity with respect to maltose or galactose is still considerably remained. Therefore, further improvement of the substrate specificity for enabling the exact measurement of an amount of glucose has been desired. In particular, PQQGDH with sufficiently low reactivity with respect to galactose has not been found and been desired to be provided.

Under such circumstances, it is an object of the present invention to provide a modified PQQGDH having high substrate specificity with respect to glucose. Inparticular, itisanobject of the present invention to provide a modified PQQGDH with extremely low reactivity with respect to galactose in addition to low reactivity with respect to maltose. Furthermore, the present invention also aims to provide polynucleotide, an expression vector and a transformant, which can be used for preparing the modified PQQGDH, and a method for preparing the modified PQQGDH using the polynucleotide, expression vector and transformant, and a kit for measuring glucose.

In order to achieve the above-mentioned objects, the present inventors have investigated in detail the relation between the substitution of amino acids in a certain region and the change in the substrate specificity in PQQGDH produced by Acinetobacter calcoaceticus. As a result, the present inventors succeeded in finding a plurality of regions deeply involved in the substrate specificity. That is to say, as mentioned in the below-mentioned Examples, the present inventors found that when the amino acid at position 351 was substituted in a wild-type PQQGDH of Acinetobacter calcoaceticus, the reactivity with respect to maltose and galactose was significantly lowered. Furthermore, the present inventors found that the amino acid at position 342 was also deeply involved in the substrate specificity. Herein, since these amino acids were located in a secondary structure including amino acid residues at positions 326 to 354, it was thought that by modifying this structural part, the substrate specificity with respect to glucose can be efficiently improved.

Furthermore, an amino acid residue at position 295 located in the secondary structure including amino acid residues at positions 278 to 320 was shown to be largely involved in the reactivity with respect to maltose and galactose. Thus, it was thought that the modification of this structural part was also effective for improving the specificity. Similarly, since it was observed that the substitution of the amino acid residue at position 169 also improved the substrate specificity, it was thought that the modification of the secondary structural part including amino acid residues at positions 162 to 197 was effective in improving the substrate specificity.

Furthermore, it was suggested that some other amino acid residues were involved in the substrate specificity.

Note here that the present inventors succeeded in obtaining various modified PQQGDHs in which amino acids were substituted in at least two or more positions 75, 168, 169, 295, 342, 347 and 351. In these modified PQQGDHs, the substrate specificity with respect to glucose was observed to be radically improved. In particular, it was clarified that in the modified PQQGDH in which amino acids at positions 169, 295, 342 and 351 were substituted had extremely low reactivity with respect to maltose and galactose. Similarly, it was clarified that the modified PQQGDH in which the amino acid at positions 342 and 351 were substituted also have extremely low reactivity with respect to maltose and galactose.

Herein, proteins having the same functions are generally similar to each other in structure of the region involved in its activity site or substrate specificity. Therefore, it is expected that the above-mentioned information as to the relation between the substitution of a certain amino acids and the substrate specificity in PQQGDH derived from Acinetobacter calcoaceticus may be applied to PQQGDH derived from other microorganisms.

The present invention was completed based on the above-mentioned findings and provides the following configurations.
[1] A modified pyrroloquinoline quinone-dependent glucose dehydrogenase, comprising an amino acid sequence in which one or more amino acids in a region corresponding to a first region consisting of amino acids at positions 326 to 354 in pyrroloquinoline quinone-dependent glucose dehydrogenase derived from Acinetobacter calcoaceticus are substituted as compared with an amino acid sequence of the corresponding wild-type enzyme.
[2] The modified pyrroloquinoline quinone-dependent glucose dehydrogenase described in [1], wherein in said region corresponding to the first region, two or more amino acids including amino acids corresponding to amino acids at positions 342 and 351 are substituted.
[3] A modified pyrroloquinoline quinone-dependent glucose dehydrogenase, comprising an amino acid sequence in which in a first region consisting of amino acids at positions 326 to 354 in the amino acid sequence of SEQ ID NO: 1, one or more amino acids are substituted by other amino acids.
[4] The modified pyrroloquinoline quinone-dependent glucose dehydrogenase described in [3], wherein in said first region, two or more amino acids including amino acids at positions 342 and 351 are substituted by other amino acids.
[5] A modified pyrroloquinoline quinone-dependent glucose dehydrogenase, comprising an amino acid sequence in which an amino acid corresponding to an amino acid at position 295 in pyrroloquinoline quinone-dependent glucose dehydrogenase derived from Acinetobacter calcoaceticus is substituted by another amino acid as compared with an amino acid sequence of the corresponding wild-type enzyme.
[6] A modified pyrroloquinoline quinone-dependent glucose dehydrogenase, comprising an amino acid sequence in which amino acids corresponding to amino acids of the following (1) and (2) in pyrroloquinoline quinone-dependent glucose dehydrogenase derived from Acinetobacter calcoaceticus are respectively substituted by other amino acids as compared with an amino acid sequence of the corresponding wild-type enzyme:
   (1) an amino acid at position 295; and
   (2) one or more amino acids selected from the group consisting of amino acids at positions 75, 168, 169, 342, 347 and 351.
[7] A modified pyrroloquinoline quinone-dependent glucose dehydrogenase, comprising any of thefollowing amino acid sequences (1) to (6):
   (1) an amino acid sequence in which amino acids corresponding to amino acids at positions 169, 295, 342 and 351 in pyrroloquinoline quinone-dependent glucose dehydrogenase derived from Acinetobacter calcoaceticus are respectively substituted by other amino acids, as compared with an amino acid sequence of the corresponding wild-type enzyme;
   (2) an amino acid sequence in which amino acids corresponding to amino acids at positions 295 and 351 in pyrroloquinoline quinone-dependent glucose dehydrogenase derived from Acinetobacter calcoaceticus are respectively substituted by other amino acids, as compared with an amino acid sequence of the corresponding wild-type enzyme;
   (3) an amino acid sequence in which amino acids corresponding to amino acids at positions 295, 342 and 351 in pyrroloquinoline quinone-dependent glucose dehydrogenase derived from Acinetobacter calcoaceticus are respectively substituted by other amino acids, as compared with an amino acid sequence of the corresponding wild-type enzyme;
   (4) an amino acid sequence in which amino acids corresponding to amino acids at positions 169, 295 and 342 in pyrroloquinoline quinone-dependent glucose dehydrogenase derived from Acinetobacter calcoaceticus are respectively substituted by other amino acids, as compared with an amino acid sequence of the corresponding wild-type enzyme;
   (5) an amino acid sequence in which amino acids corresponding to amino acids at positions 75, 295, 342 and 351 in pyrroloquinoline quinone-dependent glucose dehydrogenase derived from Acinetobacter calcoaceticus are respectively substituted by other amino acids, as compared with an amino acid sequence of the corresponding wild-type enzyme; and
   (6) an amino acid sequence in which amino acids corresponding to amino acids at positions 342 and 351 in pyrroloquinoline quinone-dependent glucose dehydrogenase derived from Acinetobacter calcoaceticus are respectively substituted by other amino acids, as compared with an amino acid sequence of the corresponding wild-type enzyme.
[8] A modified pyrroloquinoline quinone-dependent glucose dehydrogenase, comprising any of thefollowing amino acid sequences (1) to (6):
   (1) an amino acid sequence of SEQ ID NO: 1 in which amino acids at positions 169, 295, 342 and 351 are substituted by other amino acids respectively;
   (2) an amino acid sequence of SEQ ID NO: 1 in which amino acids at positions 295 and 351 are substituted by other amino acids respectively;
   (3) an amino acid sequence of SEQ ID NO: 1 in which amino acids at positions 295, 342, and 351 are substituted by other amino acids respectively;
   (4) an amino acid sequence of SEQ ID NO: 1 in which amino acids at positions 169, 295 and 342 are substituted by other amino acids respectively;
   (5) an amino acid sequence of SEQ ID NO: 1 in which amino acids at positions 75, 295, 342 and 351 are substituted by other amino acids respectively; and
   (6) an amino acid sequence of SEQ ID NO: 1 in which amino acids at positions 342 and 351 are substituted by other amino acids respectively.
[9] A modified pyrroloquinoline quinone-dependent glucose dehydrogenase, comprising any of amino acid sequences of SEQ ID NOs: 28 to 34.
[10] A modified pyrroloquinoline quinone-dependent glucose dehydrogenase, wherein the reactivity with respect to maltose is 40% or less and the reactivity with respect to galactose is 10% or less, respectively relative to the reactivity with respect to glucose.
[11] A modified pyrroloquinoline quinone-dependent glucose dehydrogenase, wherein the reactivity with respect to maltose is 20% or less and the reactivity with respect to galactose is 5% or less, respectively relative to the reactivity with respect to glucose.
[12] A modified pyrroloquinoline quinone-dependent glucose dehydrogenase, wherein the reactivity with respect to galactose is 10% or less relative to the reactivity with respect to glucose.
[13] An isolated polynucleotide encoding the modified pyrroloquinoline quinone-dependent glucose dehydrogenase described in any of [1] to [12].
[14] A vector containing polynucleotide described [13].
[15] A transformant holding polynucleotide described [13].
[16] A method for producing pyrroloquinoline quinone-dependent glucose dehydrogenase, the method comprising
   a step of culturing the transformant described in [15] in a state in which the polynucleotide can be expressed; and
   a step of separating the expressed product of the
   polynucleotide.
[17] A kit for measuring glucose, comprising the modified pyrroloquinoline quinone-dependent glucose dehydrogenase described in any of [1] to [12].

### BRIEF DESCRIPTION OF DRAWINGS

These and other objects and technical advantages of the present invention will be readily apparent from the following description of the preferred exemplary embodiments of the invention in conjunction with the accompanying drawings, in which:
Fig. 1 shows a sequence (not including the signal region) of a gene encoding pyrroloquinoline quinone-dependent glucose dehydrogenase derived from the Acinetobacter calcoaceticus and an amino acid sequence (not including the signal region).
Fig. 2 shows a sequence (including the signal region) of a gene encoding pyrroloquinoline quinone-dependent glucose dehydrogenase derived from the Acinetobacter calcoaceticus and an amino acid sequence (including the signal region).
Fig. 3 shows a structure of an expression vector pUK-GDHB(S) used in Examples.
Fig. 4 is a table showing primers used in Mutation scrambling and the amplification sites.
Fig. 5 is a table showing a mixing ratio of each DNA fragment in Mutation scrambling. The setting bias rates are made to be 0.8 for G75, G295, M342 and 351, and 0.5 for Q168, L169 and W347, wherein the setting bias rate means a rate of amino acid substitution introduced by mutation scrambling.
Fig. 6 is a table showing positions of amino acid substitution and substrate specificity in various kinds of modified amino acid substitution obtained by mutation scrambling in Examples. Reactivities with respect to various substrates are shown by relative values when the reactivity with respect to glucose is represented by a value (100).

### BEST MODE FOR CARRYING OUT THE INVENTION

The present invention provides a modified PQQGDH in which an amino acid sequence in a certain region is substituted as compared with the corresponding wild-type enzyme. The modified PQQGDH of the present invention is typically prepared or designed by substituting a part of amino acids by another amino acids in naturally occurring PQQGDH, i.e., a wild-type PQQGDH. In this way, the wild-type PQQGDH which the modified PQQGDH of the present invention is based on (derived from) is herein denoted by "the corresponding wild-type enzyme." Examples of the wild-type enzyme of the present invention include a PQQGDH produced by oxidizing bacteria such as Acinetobacter calcoaceticus, Acinetobacter baumannii, Pseudomonas aeruginosa, Pseudomonas putida, Pseudomonas fluorescens, Burkholderia cepacia, Gluconobacter oxydans, Acetobacter aceti, etc., and Agrobacterium radiobacter, Escherichia coli, Klebsiella aerogenes, and the like.

The term "corresponding" used to explain an amino acid residue or region means that between proteins (enzymes) to be compared, the functions resulting from the structure are equal to each other, and particularly means that the functions in terms of the reactivity with respect to sugars other than glucose, for example, maltose, galactose, are equal. For example, in PQQGDH derived from microorganisms other than Acinetobacter calcoaceticus, when the primary structures of the PQQGDH derived from the other microorganisms is compared with that of the PQQGDH derived from Acinetobacter calcoaceticus and the homology in the secondary structures of both PQQGDHs are taken into consideration, a region of the PQQGDH derived from the other microorganisms which is reasonably thought to have the same function as one of the secondary structure regions of PQQGDH derived from Acinetobacter calcoaceticus can be referred to as "corresponding to the secondary structure region of PQQGDH derived from Acinetobacter calcoaceticus."

Positions of the amino acids in the present specification are specified by reference numbers sequentially given from the side of the N-terminal toward the C-terminal with asparatic acid at N-terminal given the reference number 1.

In one embodiment of the modified PQQGDH according to the present invention, as compared with the amino acid sequence of the corresponding wild-type enzyme, one or more amino acids are substituted by other amino acids in a region corresponding to a first region consisting of amino acids at positions 326 to 354 in pyrroloquinoline quinone-dependent glucose dehydrogenase derived from Acinetobacter calcoaceticus (hereinafter, referred to as "A. C. PQQGDH"). The amino acid sequence of A. C. PQQGDH in the present application is a sequence specified by SEQ ID NO: 1. This sequence is an amino acid sequence of PQQGDH produced by Acinetobacter calcoaceticus IFO 12552 strain as mentioned in Examples below.

In another embodiment of the modified PQQGDH of the present invention, instead of or in addition to the above-mentioned amino acid substitution, one or more amino acids are substituted by other amino acids in a region corresponding to a second region consisting of amino acids at positions 278 to 320 in A. C. PQQGDH.

Furthermore, in a further embodiment of the modified PQQGDH, instead of or in addition to the above-mentioned amino acid substitution, in a region corresponding to a third region consisting of amino acids at positions 162 to 197 in A. C. PQQGDH, one or more amino acids are substituted by other amino acids.

Furthermore, in a further embodiment of the modified PQQGDH, instead of or in addition to the above-mentioned amino acid substitution, one or more amino acids selected from the group consisting of amino acids which respectively correspond to amino acids at positions 75, 168 to 347 in A. C. PQQGDH are substituted by other amino acids.

The number of amino acids to be substituted in each region is not particularly limited. However, it is preferable that two or more amino acids are substituted in the case of the region corresponding to the first region.

Furthermore, the position of amino acids to be substituted in each region is not particularly limited. For example, in the region corresponding to the first region, it is preferable that amino acids corresponding to the amino acids at position 342 and/or 351 in the first region are substituted. Similarly, in the region corresponding to the second region, it is preferable that amino acids corresponding to the amino acid at position 295 in the second region is substituted; and in the region corresponding to the third region, it is preferable that amino acids corresponding to the amino acids at position 75 and/or 169 in the third region are substituted.

Herein, the kinds of the amino acids after substitution are not particularly limited. For example, the amino acid at position 75 is tryptophan, the amino acid at position 168 is histidine, the amino acid at position 169 is phenylalanine, the amino acid at position 295 includes glutamic acid, asparatic acid, tyrosine, phenylalanine, etc., the amino acid at position 342 is proline, the amino acid at position 347 is arginine, and the amino acid at position 351 is threonine.

Specific examples of the modified PQQGDH of the present invention include PQQGDH having an amino acid sequence of any of SEQ ID NOs: 28 to 34. Herein, the amino acid sequence of SEQ ID NO: 28 is an amino acid sequence in which amino acids at positions 169, 295, 342 and 351 are substituted by other amino acids respectively in the amino acid sequence (SEQ ID NO: 1) of the wild-type PQQGDH produced by Acinetobacter calcoaceticus IFO12552 strain. Similarly, the amino acid sequence of SEQ ID NO: 29 is an amino acid sequence in which amino acids at positions 295 and 351 are substituted by other amino acids respectively in SEQ ID NO: 1. Similarly, the amino acid sequence of SEQ ID NO: 30 is an amino acid sequence in which amino acids at positions 295, 342 and 351 are substituted by other amino acids respectively in SEQ ID NO: 1. Similarly, the amino acid sequence of SEQ ID NO: 31 is an amino acid sequence in which amino acids at positions 295, 342 and 351 are substituted by other amino acids respectively in SEQ ID NO: 1. Similarly, the amino acid sequence of SEQ ID NO: 32 is an amino acid sequence in which amino acids at positions 169, 295 and 342 are substituted by other amino acids respectively in SEQ ID NO: 1. Similarly, the amino acid sequence of SEQ ID NO: 33 is an amino acid sequence in which amino acids at positions 75, 295, 342 and 351 are substituted by other amino acids respectively in SEQ ID NO: 1. Similarly, the amino acid sequence of SEQ ID NO: 34 is an amino acid sequence in which amino acids at positions 342 and 351 are substituted by other amino acids respectively in SEQ ID NO: 1.

As shown in the below-mentioned Examples, in the modified PQQGDH having an amino acid sequence of SEQ ID NO: 28 or an amino acid sequence of SEQ ID NO: 34, the reactivity with respect to maltose is extremely low. Furthermore, in the modified PQQGDH having an amino acid sequence of SEQ ID NO: 28, an amino acid sequence of SEQ ID NO: 29, an amino acid sequence of SEQ ID NO: 33 or an amino acid sequence of SEQ ID NO: 34, the reactivity with respect to galactose is extremely low.

The modified PQQGDH of the present invention has a feature that the selectivity with respect to glucose is improved as compared with the wild type enzyme. That is to say, the modified PQQGDH of the present invention has a feature that reactivity with respect to maltose or lactose is low and in particular the reactivity with respect to galactose is significantly low as compared with the wild type enzyme. When the reactivity is expressed by relative values respectively relative to 100% (the reactivity with respect to glucose), the reactivity of the modified PQQGDH of the present invention with respect to maltose is preferably 40% or less, more preferably 30% or less and furthermore, preferably 20% or less. Similarly, the reactivity with respect to lactose is preferably 50% or less and more preferably 40% or less. The reactivity with respect to galactose is preferably 20% or less, more preferably 10% or less, furthermore, preferably 5% or less and the most preferably substantially 0%. Note here that just "substrate specificity" herein denotes the selectivity with respect to glucose.

In the modified PQQGDH of the present invention, in addition to the above-mentioned amino acid substation, a part of amino acids may be further modified. The phrase "a part of amino acids is modified" herein is intended to include deletion or substitution of one to several amino acids constituting amino acid sequence, or addition or insertion of one to several amino acids, or the combination thereof so as to change the amino acid sequence. In principle, such modification can be carried out as long as the activity as the PQQGDH (i.e., reactivity with respect to glucose) is maintained. However, even though the activity of PQQGDH is lowered to some degree, in a case where the reactivity with respect to other substrate (for example, maltose or galactose) is also lowered, so that the substrate specificity with respect to glucose is turned out to be improved, or in a case where lowering of the substrate specificity to some degree does not influence on the measurement of an amount of glucose, modification as mentioned above are allowed.

It is preferable that the modification of a part of the amino acids as mentioned above is carried out in a state in which the reactivity with respect to maltose, galactose and the like is kept low.

Herein, the position of the amino acid to be modified is not particularly limited. Furthermore, modification may be carried out in a plurality of positions. "Plurality of positions" herein denotes 10% or less of the number of whole amino acids, and preferably 5 % or less. Furthermore preferably, it is 1% or less of the number of whole amino acids.

The modified PQQGDH of the present invention can be also prepared by firstly obtaining gene encoding a wild-type PQQGDH; then modifying the wild- type PQQGDH so as to construct polynucleotide encoding the modified PQQGDH; and finally expressing the polynucleotide in an appropriate expression system. Hereinafter, this preparing method will be described. Note here that once a sequence is designed, based on its sequence information, polynucleotide (gene) encoding the modified PQQGDH of the present invention can be prepared by chemical synthesis using deoxynucleotide triphosphoric acids (dATP, dTTP, dGTP and dCTP).

### <Obtaining gene encoding wild-type PQQGDH>

Firstly, a gene encoding PQQGDH is obtained from bacteria producing PQQGDH. Examples of the bacteria producing PQQGDH include oxidizing bacteria such as Acinetobacter calcoaceticus, Acinetobacter baumannii, Pseudomonas aeruginosa, Pseudomonas putida, Pseudomonas fluorescens, Burkholderia cepacia, Gluconobacter oxydans, Acetobacter aceti, etc.; and enterobacteria such as Escherichia coli, Klebsiella aerogenes, etc. Above all, it is preferable that Acinetobacter calcoaceticus is used as bacteria producing PQQGDH.

A gene encoding PQQGDH can be extracted from the cell body of the bacteria producing PQQGDH in the usual manner. A PQQGDH gene can also be prepared by employing PCR method, etc. using the genome DNA of PQQGDH as a template. On the other hand, an extracted gene is amplified by using an amplification method such as PCR method, and then the amplification product may be used in the following operations. Note here that after the base sequence of the gene encoding PQQGDH is identified, the targeted PQQGDH gene can be prepared also by a chemical technique. Hereinafter, as one example of preparing a PQQGDH gene, a case where the preparation using Acinetobacter calcoaceticus IFO 12552 strain as a starting material will be described in detail.

Firstly, a DNA fragment obtained by isolating and purifying a chromosome of Acinetobacter calcoaceticus IFO 12552 and thereafter subjecting it to an ultrasonication treatment, a restriction enzyme treatment, etc. and an expression vector prepared in a linear state are ligated to each other at their both blunt ends or sticky ends so as to be closed with the use of DNA ligase, etc. Thus, a recombinant vector is constructed. A recombinant vector can be also constructed by amplifying the PQQGDH gene by a polymerase chain reaction (PCR) using an oligonucleotide primer designed based on the base sequence of the well-known PQQGDH derived from Acinetobacter species L.M.D 79.41 strain and ligating thereof with the use of DNA ligase. Then, the recombinant vector that was constructed by any of the above-mentioned methods is transferred into a host microorganism in which the vector can autonomously replicate. Then, screening is carried out using a marker unique to an expression vector and PQQGDH activity as indices, and thus a host microorganism transformed by a recombinant vector containing a gene encoding PQQGDH is obtained.

After the selected transformant is cultured if necessary, cell body is collected. A recombinant vector is isolated and purified from the collected cell body in the usual manner. Thus, a recombinant vector containing the targeted PQQGDH gene is obtained. The isolation of the PQQGDH gene from the recombinant vector can be carried out by restriction enzyme treatment, etc.

Herein, one example of obtaining PQQGDH gene from Acinetobacter calcoaceticus IFO 12552 is described in detail. First of all, a culture medium, which is obtained by culturing Acinetobacter calcoaceticus IFO 12552 with stirring for, e.g. 1 to 3 days, is centrifuged so as to collect bacterial cells. Then, the collected cells are dissolved so as to prepare a lysate containing PQQGDH gene. As a method for dissolving bacterial cells, for example, treatment using a lysatic enzyme such as lysozyme can be employed. Furthermore, if necessary, enzymatic treatment with protease, etc. or treatment with a surface active agent such as sodium lauryl sulfate (SDS) may be carried out together. Furthermore, physical crushing method such as freezing and thawing or French press treatment may be combined.

DNA can be isolated and purified in the usual manner from the lysate obtained as mentioned above. For example, it can be carried out by appropriately combining methods such as deproteinization treatment by phenol treatment or protease treatment, ribonuclease treatment, alcohol precipitation, and the like.

In order to obtain PQQGDH gene from DNA isolated and purified from microorganism, a polymerase chain reaction using a primer based on the base sequence of Acinetobacter species L.M.D 79.41 strain, which was already specified, is carried out.

Also, the amplification PQQGDH gene can be cloned in an appropriate vector. As a vector to be cloned (cloning vector), a vector constructed as a recombinant vector from a phage or plasmid capable of autonomously replicating in a host microorganism is suitable. Examples of such a phage include Lambda gt 10, Lambda gt11, etc. using Escherichia coli as a host organism. Similarly, examples of such a plasmid include pBR322, pUC19, pBluescript, pTrc99A, pGEM-T vector, etc. using Escherichia coli as a host organism.

Cloning into a vector can be carried out by inserting PQQGDH gene obtained in the above-mentioned method into a cloning site. Such an operation can be carried out by a well-known method using a restriction enzyme and DNA ligase.

The host microorganism to be used for cloning is not particularly limited as long as a recombinant vector is capable of growing stably and autonomously and expressing an exogenous gene. In general, examples of such host microorganisms include Escherichia coli W3110, Escherichia coli C600, Escherichia coli HB 101, Escherichia coli JM109, Escherichia coli DH5α, etc.

As a method for transferring a recombinant vector into a host organism, for example, in the case where, for example, the host organism is Escherichia coli, a competent cell method using a calcium treatment, electroporation, etc. can be used.

In order to select a host microorganism into which a targeted recombinant vector is introduced appropriately, a microorganism capable of expressing a marker (for example, drug resistant gene) of a vector and capable of expressing GDH activity by the addition of PQQ may be searched. For example, a microorganism which grows in a selective medium based on a drug resistant marker and produces PQQGDH may be selected. The transformant selected in this way can produce a large amount of PQQGDH stably when it is cultured in a nutritional medium.

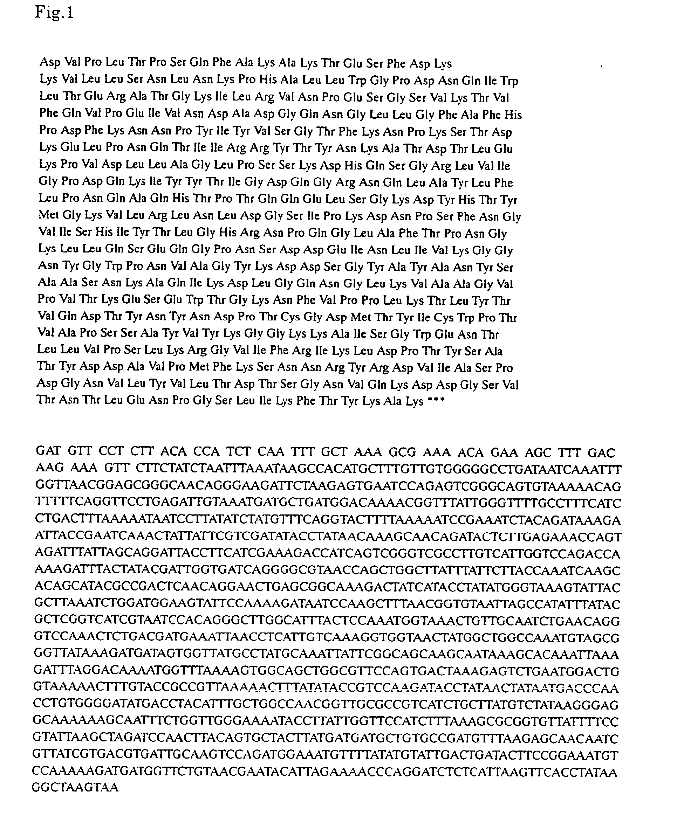

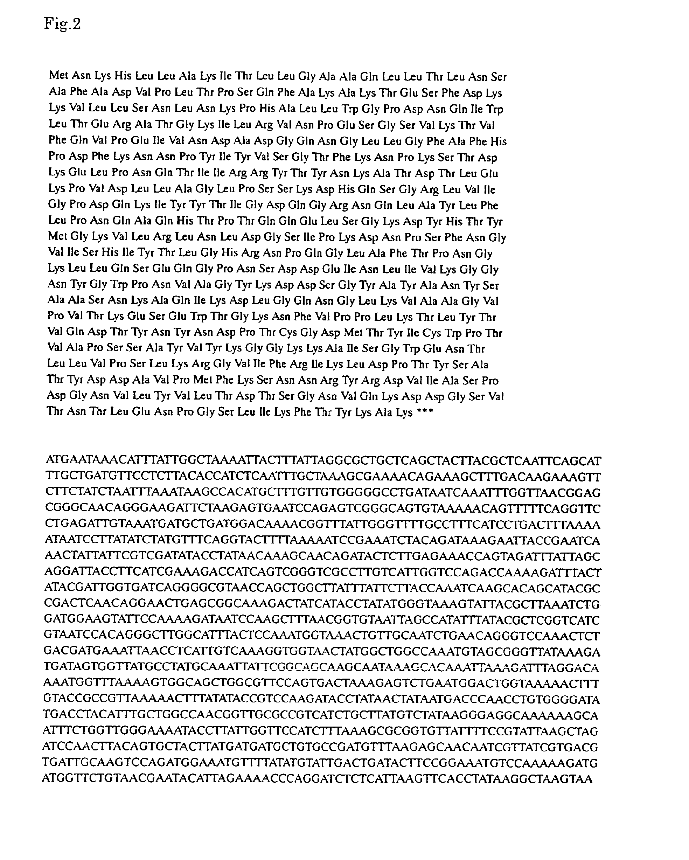

A base sequence of a wild-type PQQGDH gene obtained by the above-mentioned method is decoded by the dideoxy method described in Science, vol. 214, 1205 (1981) (see Fig. 1, SEQ ID NO: 2). Furthermore, the sequence of amino acids of PQQGDH was estimated by the base sequence determined as mentioned above (see Fig. 1, SEQ ID NO: 1). Note here that gene sequence (SEQ ID NO: 4) and amino acid sequence (SEQ ID NO: 3), which include the signal region, are shown in Fig. 2.

The PQQGDH gene collected from the recombinant vector held by the PQQGDH gene can be easily modified by a genetic engineering technique. Specifically, for example, gene is modified so that amino acid residues in a certain site include substitution, deletion, insertion, addition or inversion.

### <Construction of polynucleotide encoding modified PQQGDH>

By adding mutation to PQQGDH gene obtained in this way so that certain amino acids are substituted in an expression product, genes encoding the targeted modified PQQGDH can be prepared. A large number of methods for carrying out the site specific substitution of base sequence are known in this technical field (see, for example, Molecular Cloning, Third Edition, Cold Spring Harbor Laboratory Press, New York) and therefore, an appropriate method can be selected from such methods. Note here that also a random mutation is inserted into PQQGDH so as to produce mutation products, and then the substrate specificities of the expression products of the mutation products (modified PQQGDHs) are compared to each other. Thereby, by selecting genes having a preferable substrate specificity, gene encoding the modified PQQGDH can be prepared. In a case where such a random mutation is inserted, firstly, mutation is inserted into a targeted gene region at random by using, for example, an error-prone PCR, and thus modified PQQGDH gene library is constructed. A multiple substitution containing a combination of useful mutations obtained by random mutation can be obtained by a mutation scrambling method. The mutation scrambling method was reported by Norvartis Pharma in Journal of Bioscience and Industry, vol. 59, No. 3 (2001), P35-38. In this method, the modified enzyme can be evaluated simply and easily by a procedure including culturing the resultant colonies, preparing lysate of bacteria by the use of a lysate enzyme such as lysozyme, and measuring the enzymatic activity.

In order to screen the modified PQQGDH gene library efficiently, a base sequence encoding a histidine tag may be added into a PQQGDH gene in advance. Thus, despite of the change in an amount of production enzyme due to a growing state and expression efficiency, etc., enzyme activity can be evaluated by using a certain amount of enzyme. Specifically, for example, a certain amount of anti-histidine tag monoclonal antibody is adsorbed onto the surface of the substrate such as a microplate having a property of adsorbing protein, a treatment for inhibiting non-specific proteins from being adsorbed thereon is carried out, followed by reacting it with the above-mentioned lysate. Thus, a certain amount of PQQGDH contained in the above-mentioned lysate is made into a solid phase, and the enzyme activity can be evaluated. By carrying out the above-mentioned operation in a 96-well microplate , the enzyme activity of the unit amount of a large number of modified PQQGDHs can be evaluated at one time efficiently.

For example, a clone holding the targeted modified gene is selected as follows. Based on the absorbance at 410 nm one hour after 1-methoxy-PMS and XTT are added into lysate, the activity of PQQGDH is measured. At this time, the sample whose reactivity with respect to maltose is lowered to about 80% or less as compared with the wild-type PQQGDH is firstly selected, and then a clone whose reactivity with respect to glucose is maintained at about 90% or more as compared with the wild-type PQQGDH is selected. Herein, the base sequence of the resultant clone is analyzed so as to confirm the mutation.

### <Expression of modified PQQGDH gene>

For expression of the modified PQQGDH gene, an expression system using Escherichia coli as a host can be used. For example, firstly, the modified PQQGDH gene prepared by the above-mentioned method is inserted into a vector using Escherichia coli as a host (for example, a pUC vector, a pBluescript vector) so as to construct an expression vector. The expression vector includes a promoter sequence necessary for expressing the modified PQQGDH in the host, a replication origin, a terminator sequence, and the like.

As a method for introducing (transferring) an expression vector into a host microorganism, a competent cell method using a calcium treatment, electroporation, etc. can be used. Selection of host microorganism into which an expression vector can appropriately introduced can be carried out by using the presence or absence of a drug resistant marker and the presence or absence of expression of the GDH activity when PQQ is added as indices. For example, the microorganism after introducing of an expression vector is cultured in a selection medium capable of growing only in the case where it has drug resistant marker, and then among transformants observed to be grown, a transformant capable of producing a modified PQQGDH may be selected.

By culturing the transformant selected in the above-mentioned operation under conditions where it can be grown and proliferated and the modified PQQGDH gene inserted into an expression vector can be expressed, a large amount of modified PQQGDHs can be produced stably.

As a nutrient of a medium, nutrient that can usually be used for culturing microorganism can be used. As a carbon source, a carbon compound capable of assimilation may be used. For example, glucose, sucrose, lactose, maltose, lactose, syrup, pyruvic acid, and the like can be used. Furthermore, as a nitrogen source, a usable nitrogen compound can be used. For example, peptone, meat extract, yeast extract, casein hydrolysate, soybean cake alkaline extract, and the like, can be used. Besides, salts such as phosphate, carbonate, sulfate, magnesium, calcium, potassium, iron, manganese, zinc, etc. , certain amino acids, certain vitamin, and the like, can be used if necessary.

Transformant is cultured under temperature conditions where the transformant can be grown and modified PQQGDH can be produced. For example, the culture temperature can be set at 20° C to 30°C. The culturing period can be set in consideration of the growing property of the transformant to be cultured, the production property of the modified PQQGDH, or the necessary production amount of the modified PQQGDH and the like. For example, at the time when the yield of the modified PQQGDH reaches maximum, the culture is completed. The culturing period may be about 12 to 72 hours. pH of the medium is adjusted to the range in which the transformant can grow and PQQGDH can be produced. Preferable pH is about 6.0 to 9.0.

The culture medium containing cell bodies producing modified PQQGDH can be used as they are or used after they are subjected to treatment such as concentration treatment or treatment for removing impurities, etc. However, in general, the modified PQQGDH is collected from the culture medium or cell bodies. When the produced modified PQQGDH is a secretion type protein, it can be collected from the culture medium; and the produced modified PQQGDH is other than the secretion type protein, it can be collected from the cell bodies. In case that the modified PQQGDH is collected from the culture medium, for example, culture supernatant is filtrated, subjected to centrifugal treatment so as to remove impurities, followed by carrying out vacuum concentration, membrane concentration, salting out using ammonium sulfate or sodium sulphate, fractional precipitation method by using methanol, ethanol, acetone, or the like, dialysis, heat treatment, isoelectric treatment, various kinds of chromatography such as gel filtration or adsorption chromatography, ion exchange chromatography, affinity chromatography, etc. (for example, gel filtration by using Sephadex gel (Pharmacia Biotech), etc., DEAE sepharose CL-6B (Pharmacia Biotech), octyl sepharose CL-6B (Pharmacia Biotech), CM sepharose CL-6B (Pharmacia Biotech)), and the combination thereof so as to isolate and purify modified PQQGDH product. Thus purified product of the modified PQQGDH can be obtained. On the other hand, when the modified PQQGDH is collected from cell body, by carrying out the filtration and centrifugal treatment, etc. of culture medium, cell bodies are collected, and then the cell bodies are crushed by a mechanical method such as a pressure treatment, an ultrasonic treatment, etc. or by an enzymatic method using lysozyme etc. Thereafter, similar to the above, isolation and purification is carried out so as to obtain a purified product of the modified PQQGDH. Note here that if necessary, by adding a chelating agent such as EDTA and a surface active agent, PQQGDH may be solubilized and PQQGDH can be isolated and collected as an aqueous solution.

It is preferable that the purified enzyme is purified to such an extent that it shows a single band in an electrophoresis (SDS-PAGE).

The purified enzyme obtained as mentioned above can be distributed in a state of particles by, for example, freeze-drying, vacuum drying or spray drying, and the like. At this time, the purified enzyme may be dissolved in a phosphate buffer solution, a TRIS hydrochloric acid buffer solution, or Good's buffer solution in advance. The suitable buffer solution to be used herein is a Good's buffer, and above all, PIPES, MES or MOPS buffer solution is particularly preferable.

The modified PQQGDH of the present invention catalyzes a reaction for producing δ-gluconolactone by oxidizing glucose using PQQ as a coenzyme. Such an enzyme activity can be determined by measuring an amount of PQQ reduced in accordance with oxidization of glucose by PQQGDH by a color reaction with a redox reagent. As the color reagent, for example, PMS-DCIP, 1-methoxy-PMS-XTT, potassium ferricyanide, and the like can be used.

In the present invention, in principle, the PQQGDH activity can be measured by using the following reagent under the following conditions.

### <Reagent>

10 mM MOPS (pH 7.0)
1 mM 1-methoxy PMS (phenazine methosulfate)
0.25 mM XTT
5 mM glucose

### <Measurement conditions>

140 µl of the above-mentioned reagent mixing solution is preheated at 25° C for about 5 minutes, followed by adding 0.1 ml of enzyme solution to calmly admix thereof. Thereafter, the admixture is subject to recordation for 15 minutes by a spectrophotometer controlled at 25° C with water as a control and from the linear part, change in absorbance at 410 nm for 1 minute is measured. In a blind test, instead of enzyme solution, distilled water is added in a test mixture, and change in absorbance of light with 410 nm for 1 minute is similarly measured. In the above-mentioned conditions, the amount of enzyme of 1/2 µmol of formazan produced per minute is made to be 1 unit (U).

The selectivity with respect to glucose of the modified PQQGDH of the present invention can be evaluated as follows. That is to say, enzyme activities when the substrate are various kinds of sugars such as lactose, maltose, galactose, sucrose and xylose are examined in the similar way to the above. The resultant enzyme activities are expressed as relative values relate to the activity when the substrate is glucose. Thus, the relative activities can be evaluated.

By using the modified PQQGDH of the present invention, it is possible to construct a glucose measurement kit. That is to say, another aspect of the present invention provides a glucose measurement kit containing the modified PQQGDH. In addition to the modified PQQGDH, such a kit can contain a solution such as a buffer solution and a glucose solution as a standard, etc. which are necessary for measurement.

The following examples further illustrate the present invention. These examples are not intended to limit the scope of the present invention.

### [Example 1] Isolation of chromosomal DNA

Chromosomal DNA of Acinetobacter calcoaceticus IFO 12552 strain was isolated by the following method. This strain was cultured in 10 mL of LB medium with shaking at 30° C over night, and then centrifuged (at 15000 rpm for 10 minutes) so as to collect bacteria. From the collected bacteria, chromosomal DNA was extracted and purified by using a Dneasy tissue kit (QIAGEN K.K.) and dissolved in a TE buffer.

### [Example 2] Preparation of DNA fragment containing gene encoding PQQGDH, and Construction of recombinant vector containing DNA fragment.

The chromosomal DNA obtained in Example 1 was used as a template to amplify a region of DNA including the targeted PQQGDH gene by polymerase chain reaction (PCR) using the following primers. Note here that the primers to be used herein were designed based on a base sequence of soluble PQQGDH (A-M Cleton-Jansen et al., Gen. Genet., 217, 430 (1989)) derived from Acinetobacter species L.M.D 79.41 strain.

Forward primer:

5′-ACAAATCATATAGAGAACTCG-3′ (SEQ ID NO: 5)

Reverse primer:

5'-TTACTTAGCCTTATAGGTGAACTTAATGAGAGATCCTGGG-3'

(SEQ ID NO: 6)

The PCR was carried out in a solution having the composition shown in Table 1 under conditions of carrying out a reaction at 94°C for 2 minutes, then 30 cycles of reactions at 94°C for 30 seconds, at 48°C for 30 seconds and at 72°C for 2 minutes, and finally a reaction at 72°C for 10 minutes.

**[Table 1]**

| |
|---|
| TAKARA LA-taq: 0.5 µL |
| 10-fold buffer: 5 µL |
| 25mM MgCl₂: 5 µL |
| dNTP mix (2.5mM): 8 µL |
| Forward primer (10 pmol/pL): 1 µL |
| Reverse primer (10 pmol/pL): 1 µL |
| template |
| adjusted to 50 µL by H₂O |

The resultant amplification gene fragment was ligated to a pGEM-T Easy vector (QIAGENK.K.) and Escherichia coli JM109 strain was transformed in this plasmid. Note here that the resultant plasmid was defined as pTGEM-GDHB.

### [Example 3] Determination of sequence list of soluble PQQGDH of Acinetobacter calcoaceticus IFO 12552 strain

The sequence of the gene fragment inserted into the plasmid pTGEM-GDHB obtained in Example 2 was determined by the use of BigDye Terminator Sequencing Kit (Applied Biosystems Japan Ltd.). The determined base sequence and amino acid sequence are shown in SEQ IDNO: 2 and SEQ ID NO: 1, respectively. Note here that base sequence and amino acid sequence including the signal regions are shown in SEQ ID NO: 4 and SEQ ID NO: 3, respectively. The molecular weight of protein calculated from the amino acid sequence was about 50,000 which was substantially identical with the molecular weight of soluble PQQGDH of Acinetobacter calcoaceticus.

### [Example 4] Construction of expression vector containing PQQGDH gene

The plasmid pTGEM-GHDB obtained in Example 2 was used as a template and polymerase chain reaction (PCR) using the following primers was carried out.

Forward primer: 5′-GCGGCCGCGAATTCATGAATAAACATTTATTGGCTA

AAATTACTTTAT-3′ (SEQ ID NO: 7)

Reverse primer: 5'-GCGGCCGCCTGCAGCTATTACTTAGCCTTATAGGTG
AACTTAATGAGAGATCCTGGG-3' (SEQ ID NO: 8)

The PCR was carried out in a solution having the composition shown in Table 2 under conditions of carrying out a reaction at 94° C for 2 minutes, then 30 cycles of reactions at 94° C for 30 seconds, 55° C for 30 seconds and 72° C for 2 minutes, and finally a reaction at 72°C for 10 minutes.

**[Table 2]**

| |
|---|
| Sigma KlenTaq: 0.5 µL |
| 10-fold buffer: 5 µL |
| dNTP mix (10 mM): 2.5 µL |
| Forward primer (10 pmol/µL): 2 µL |
| Reverse primer (10 pmol/µL): 2 µL |
| template |
| adjusted to 50 µL by H₂O |

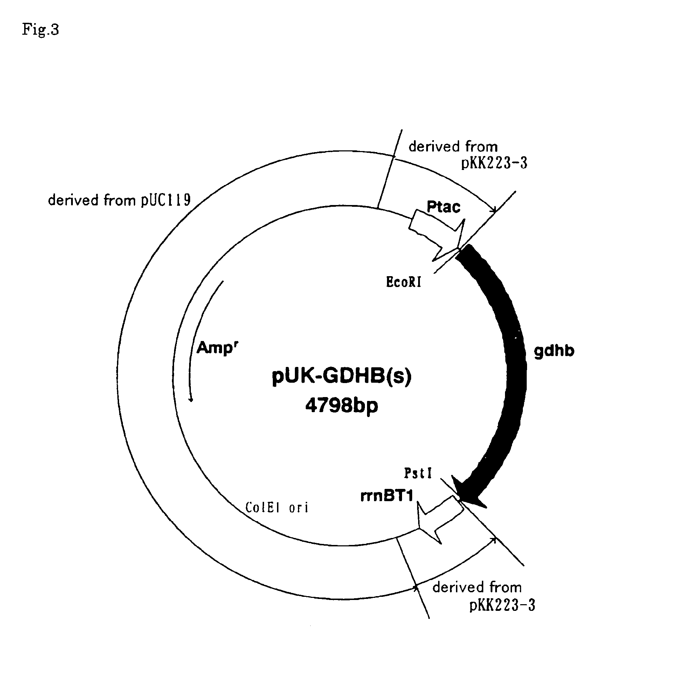

The resultant amplification gene was cut by restriction enzymes EcoRI and PstI. The cut gene fragment was ligated to an expression vector. Escherichia coli JM109 strain was transformed with this plasmid. Note here that the resultant expression plasmid was defined as pUK-GDHB(S) (see Fig. 3). When the transformant was cultured in 100 mL of LBmedium containing 100 µg/mL of ampicillin and 6 µM PQQ at 30° C over night, production of PQQGDH was observed. Note here that the molecular weight of this PQQGDH calculated from the amino acid sequence was about 50,000 which was substantially identical to the molecular weight of Acinetobacter calcoaceticus IFO 12552.

### [Example 5] Production of Acinetobacter calcoaceticus PQQGDH gene product

50 mL each of LB medium was dispensed in a 500 mL vol flask and subjected to autoclaving at 121° C for 20 minutes. Aftercooling down, to the medium, 100 mg/mL ampicillin solution and isopropyl galactopyranoside (50 µL), which were separately filtrated aseptically, were added and a colony having a PQQGDH expression vector pUK-GDHB(S) was inoculated. Then, the medium was cultured with shaking at 30° C for 16 hours. When the culture was completed, the PQQGDH activity was about 1 U/mL.

After culturing, the medium was centrifuged so as to collect bacteria. The collected bacteria were suspended in 10 mM Tris-HCL (pH 7.5). Then, the bacteria suspension was ultrasonic disintegrated and then centrifuged again so as to obtain a supernatant as a crude enzyme solution. This crude enzyme solution was treated with a cationic coagulation agent and concentrated by desalting, followed by separating and purifying by a DEAE sepharose (Pharmacia Biotech), CM sepharose (Pharmacia Biotech) column chromatography. Thus, purified enzyme was obtained.

The PQQGDHs obtained by the above-mentioned method showed a substantially a single band in electrophoresis. The specific activity at this time was about 1600 U/mg. Hereinafter, properties of the resultant PQQGDH are described.
Action: D-glucose + artificial electron acceptor →d-gluconolacton + reduced electron acceptor
Thermal stability: 50° C (pH: 7.0, treatment for 10 minutes) pH stability: pH 6 to 9 (30°C, treatment for one hour)
Optimum temperature: about 50°C
Optimum pH: 7.0
Molecular weight: 50,000

### [Example 6] Addition of C-terminal histidine tag of PQQGDH Firstly, a histidine tag was introduced in the downstream of PQQGDH gene. Then, a PCR was carried out using a primer for adding a stop codon right behind it. A PCR using the following primers was carried out using pUK-GDHB(S) obtained in Example 4 as a template.

Forward primer: 5'-TCACATGTTCTTTCCTGCGTTATC-3' (SEQ ID
NO: 9)

Reverse primer: 5'-ATGGTGATGGTGATGGTGCTTAGCCTTATAGGTGAA
CTTAATGAGA-3' (SEQ ID NO: 10, underline is given to the sequen
ce of 6 × histidine tag)

The PCR was carried out under the same conditions as in Example 4.

Then, the PCR using the following primers was carried out in order to introduce a translation stop codon in the downstream of a histidine tag by using an amplified gene as a template.

Forward primer: 5'-TCACATGTTCTTTCCTGCGTTATC-3' (SEQ ID
NO: 9)
Reverse primer: 5'-GCGGCCGCCTGCAGCTATTAATGGTGATGGTGCTTA
GCCTTA-3' (SEQ ID NO: 11)

The PCR was carried by using the same solution composition and under the same conditions as in Example 4.

The amplified gene obtained by adding a histidine tag and a stop codon in the downstream of PQQGDH was cut with EcoRI and PstI. The cut gene fragment was ligated to an expression vector so as to construct a plasmid. Escherichia coliJM109 strain was transformed with this plasmid. Note here that the resultant expression plasmid was defined as pUK-GDHB(S)C-His. When the transformant was cultured in 100 mL of LB medium containing 100 µg/mL of ampicillin and 6 µM PQQ at 30° C over night, the production of PQQGDH was observed. Note here that the molecular weight of this PQQGDH calculated from the amino acid sequence was about 50,000 which was substantially identical to the molecular weight of Acinetobacter calcoaceticus IFO 12552. Furthermore, when general properties were compared with the wild-type PQQGDH obtained in Example 5 as a control, they were found to be equal to each other.

### [Example 7] Construction of mutant PQQGDH gene library

The plasmid pUK-GDHB(S)C-His obtained in Example 6 was used as a template to introduce random mutations by error-prone PCR using the following primers.

Forward primer: 5'-TCACATGTTCTTTCCTGCGTTATC-3' (SEQ. ID
NO: 9)

Reverse primer: 5'-GGCGCGTACTATGGTTGCTTTGA-3' (SEQ ID N
O: 12)

The conditions for introducing mutations are mentioned below. The PCR was carried out in a solution having the composition shown in Table 3 under conditions of carrying out a reaction at 94 °C for 5 minutes, then 25 cycles of reactions at 94° C for one minute, 50°C for one minute and 70°C for 2 minutes, and finally a reaction at 72°C for 7 minutes.

**[Table 3]**

| |
|---|
| 100 mM Tris-HCl(pH8.3): 0.5 µL |
| 500 mM KCl: 5 µL |
| 1 mg/mL BSA: 2.5 µL |
| (15-100 mM) MgCl₂: 5 µL |
| 1 mM MnCl₂: 1.25 µL to 3.75 µL |
| 10 mM ATP: 0.5 µL to 1.55 µL |
| 10 mM TTP: 0.5 µL to 5.0 µL |
| 10 mM GTP: 1.0 µL to 5.0 µL |
| 10 mM CTP: 0.5 µL to 5.0 µL |
| Forward primer (20 pmol/µL): 2.5 µL |
| Reverse primer (20 pmol/pL): 2.5 µL |
| Dimethylsulfoxide: 0 to 7.5 µL |
| 0.2 ng/µL template: 5 µL |
| rTaq Polymerase: 0.5 µL |
| adjusted to 50 µL by H₂O |

The amplified gene fragment into which the mutation was introduced was cut with EcoRI and PstI so as to be substituted by the wild-type gene of pUK-GDHB(S). Escherichia coli JM109 was transformed with the thus obtained plasmid so as to construct a mutant gene library.

The resultant colony was inoculated into 600 µL of Circle Grow Medium (Stratagene) in a 96-well culturing microplate having a deep well. The Circle Grow Medium contained 100 µg/mL ampicillin, 5 µM of PQQ and 1 mM calcium chloride. The medium was cultured with shaking at 200 rpm at 37° C over night. Thereafter, bacteria were collected through a centrifuge (at 3000 rpm, 4° C, 30 minutes) and suspended in 600 µL of three-times diluted lysate (50 mM TRIS buffer solution, pH 8.0, 2 mM EDTA, 1 mg/mL lysozyme, 15 µM PQQ) again. Then, the suspension was subjected to shaking at 37° C for one hour and 30 µL of 20 mM calcium chloride solution was added therein. The lysate was transferred to a filter plate (Corning) and centrifuged (at 2000 rpm, at 4 ° C for 5 minutes). The resultant supernatant was used for screening mentioned below.

A 96-well microplate to be used for screening was treated in the following method in advance. That is to say, firstly, 100 µL of 15 ng/µL Protein A/G solution was added in a 96-well microplate (Coaster Co., Ltd.) and allowed it stand at 37° C for about 2 hours, then washed with a PBS solution three times, added by 150 µL of a blocking solution and allowed it stand at 37° C for about 2 hours. Thus, a microplate for screening was prepared. Into a 96-well microplate containing 80 µL of 0.1 ng/µL mouse IgG1 anti 5×histidine tag monoclonal antibody was prepared in advance, the above-mentioned supernatant was added and admixed, followed by adding into a screening microplate from which 100 µL of blocking solution was washed to be removed with a PBS solution and allowed it stand 25° C for 1 hour. Thereafter, the microplate was washed with PBS three times and added by 140 µL of PQQGDH assay solution (mixing solution containing 3.5 mL of 4 mM 1-methoxy PMS, 3.5 mL of 1mM XTT solution, 3.5 mL of 20 mM maltose or glucose solution, 3.5 mL of 40 mM MOPS-NaOH, pH 7.0) and the absorbance at 410 nm was measured at room temperature for 15 minutes.

In the first stage of the screening, a random one-base substitution library was screened with maltose as a substrate so as to identify a clone for expressing a mutant whose enzyme activity was lowered by about 80% or less of that of the wild-type enzyme. The identified mutant strain was screened so as to obtain a clone expressing a mutant whose reactivity with respect to maltose is lower than that with respect to glucose. When the base sequence of these mutant PQQGDH genes was determined, it was clarified that glycine at position 75 was mutated to arginine, glycine at position 295 was mutated to glutamic acid, methionine at position 342 was mutated to valine; alanine at position 351 was mutated to threonine; glutamine at position 168 was mutated to histidine; leucine at position 169 was mutated to glutamine; and tryptophan at position 347 was mutated to arginine. As shown Table 4 below, the modified PQQGDH had lowered specificity to maltose as compared with that of the wild-type. The activity of the wild-type PQQGDH using glucose as a substrate was made 100, and with respect to this, the reactivity with respect to maltose was shown. Note here that as a control, a wild-type PQQGDH added by a histidine tag obtained in Example 6 was used.

In Table 4, numbers in the column of amino acid substitution represent amino acid number and letters written at the left of the numbers are kinds of amino acids in a wild-type and letters at the right of the numbers are kinds of amino acids after being mutated. Therefore, for example, Gly75Arg means a modified product in which glycine that is an amino acid at position 75 was mutated to arginine.

**[Table 4]**

| Amino acid substitution | glucose | Maltose |
|---|---|---|
| Wild-type | 100 | 97 |
| Gly75Arg | 100 | 72 |
| Gln168His | 100 | 55 |
| Lue169Gln | 100 | 63 |
| Gly295Glu | 100 | 83 |
| Met342Val | 100 | 69 |
| Trp347His | 100 | 85 |
| Ala351Thr | 100 | 59 |

### [Example 8] Introduction of site-specific mutation into wild-type PQQGDH gene and consideration of substrate specificity of modified PQQGDH

A site-specific mutation was introduced into a structural gene of PQQGDH derived from Acinetobacter calcoaceticus shown by SEQ ID NO: 4 by using a QuikChange Site-directed Mutagenesis kit (Stratagene). The introduction of mutation was carried out under conditions where a base sequence encoding the amino acids at sites into which the mutation was introduced in Example 7 , that is, glycine at position 75, glycine at position 295, methionine at position 342, alanine at position 351, glutamine at position 168, leucine at position 169 and tryptophane at position 347 are substituted so as to encode 20 kinds of amino acids at random. The sequences of primers for substituting the sites by 20 kinds of amino acids are shown in Table 5. Note here that in Table 5, F and R which are given to the end sequence names represent forward and reverse, respectively.

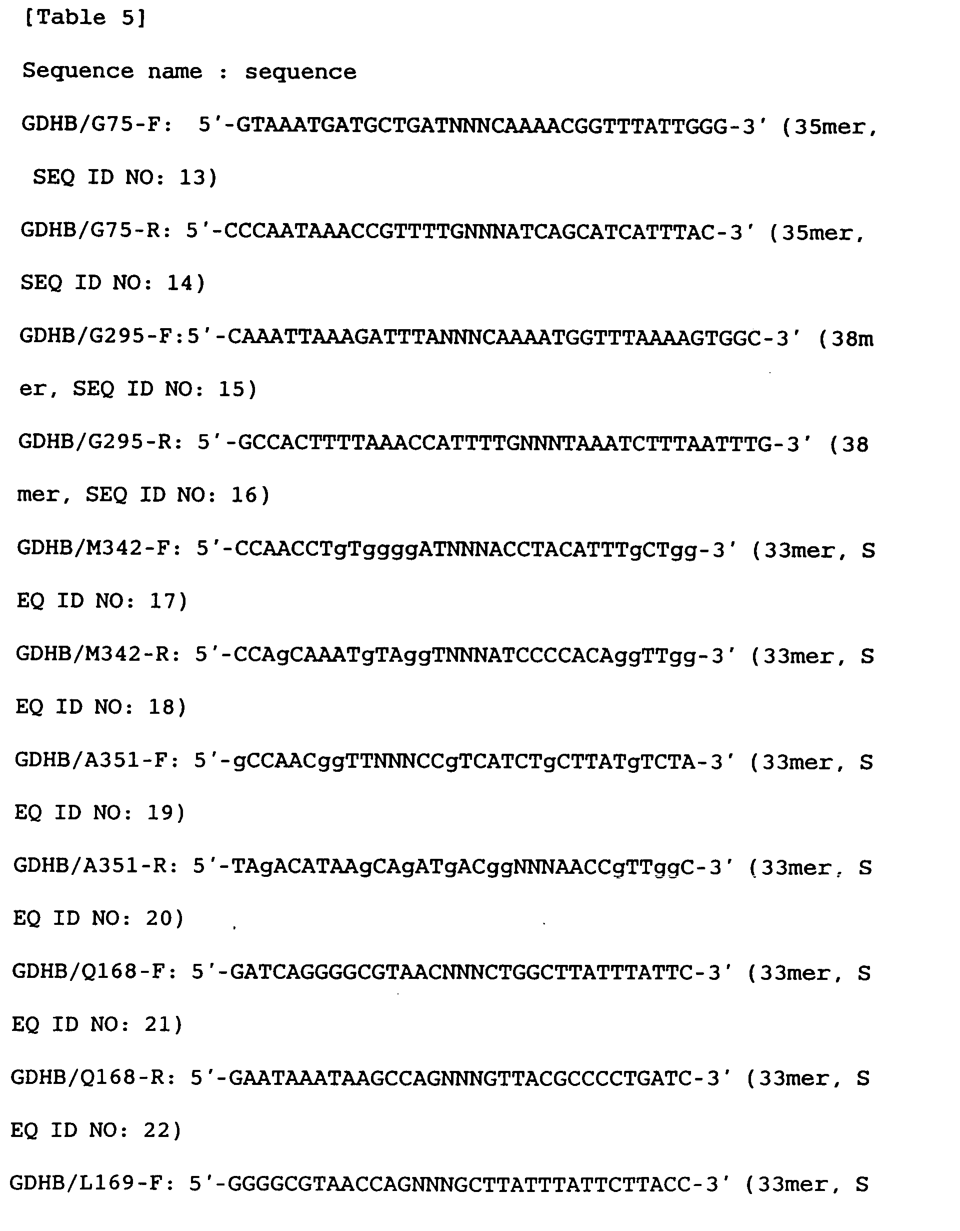

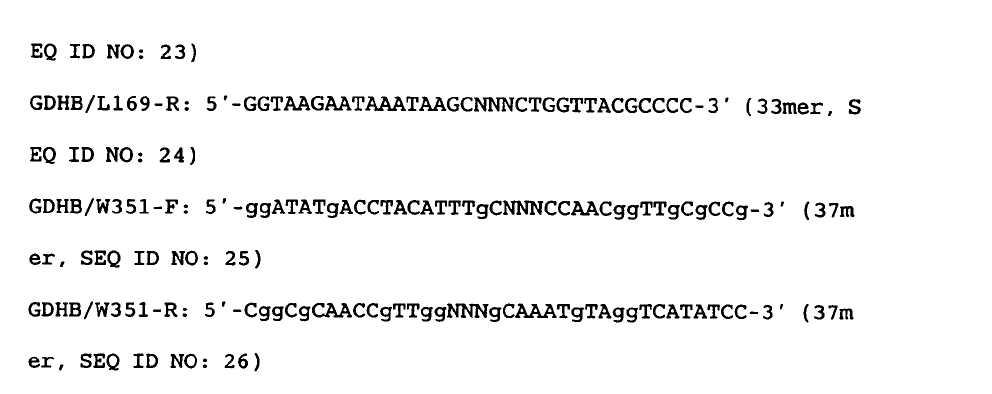

1 µL of PfuTurbo DNA polymerase (2.5 U/pL) attached to a QuikChange Site-directed Mutagenesis kit, 125 ng of forward and reverse primers, mixture of dNTP, 10 ng of pUK-GDHB(S) and 1/10 amount of a PCR buffer were mixed. The reaction was carried out under conditions for carrying out a reaction at 95° C for 30 seconds, and then 16 cycles of reactions at 95° C for 30 seconds, 55° C for 1 minute and 68° C for 10 minutes. After the reaction, in order to remove a template DNA, 1 µL of restriction enzyme DpnI (10 U/µL) was added, followed by incubation at 37° C for one hour. Escherichia coli XL1-Blue was transformed by using 1 µL of the reaction product and thus a colony was obtained.

Then, a colony of the resultant transformant was inoculated in 600 µL of Circle Grow medium (Stratagene) and the screening was carried out by the same method as Example 7. As a result, when the base sequence of the selectedmutant strain was determined, it was found that various kinds of mutants in which amino acids are substituted as shown in following Table 6. On the other hand, each of these mutants was cultured in 2 mL of LB medium containing 100 µg/mL of ampicillin, 0.01 mM isopropyl thiogalactoside and 6 µM PQQ at 30°C over night. After being cultured, bacteria were collected by a centrifuge and suspended again in 500 µL of 10 mM MOPS buffer containing 6 µM PQQ (pH 7.0). Then, 250 mg of glass beads (Yasui Kikai Corporation) were added so as to crush the cell bodies by a multibeads shocker (Yasui Kikai Corporation) (crushing operation was carried out 7 cycles of operation for 60 seconds and interval for 30 seconds).

The solution in which cell bodies were crushed was subjected to be centrifuged (15000 rpm, for 10 minutes, at 4°C) and the supernatant was purified by using His MicroSpin Purification Module (Amersham Pharmacia Biotec). As a result , 200 µL of purified enzyme solution was obtained. Into this purified enzyme solution, PQQ was added to be 5 µM and allowed to stand in the presence of 1 mM calcium chloride for 1 hour or more so as to make it holo.

The substrate specificity of each modified PQQGDH obtained as mentioned above was measured by the following method. That is to say, 50 µL each of the purified enzyme solution was dispersed and 130 µL of assay solution (60 µL of 20 mM solution of 3.3 mM 1-methoxy PMS dissolved in 20 mM MOPS (pH 7.0) and 70 µL of 171 µM XTT) and 20 µL of D-glucose solution was added so that the final concentration became 5 mM. The PQQGDH activity was measured in accordance with the method shown in Example 5. Note here that as a control, a wild-type PQQGDH added by a histidine tag obtained in Example 6 was used. The measurement results are shown in Table 6. As is apparent from Table 6, as compared with the wild-type PQQGDH, any of the modified PQQGDHs have lowered reactivity with respect to both maltose and galactose. Furthermore, at the same time, the modified PQQGDH having lowered reactivity with respect to lactose and galactose was obtained. Note here that in Table 6, numbers in the column of amino acid substitution represent amino acid number and letters written at the left of the numbers are kinds of amino acids in a wild-type and letters at the right of the numbers are kinds of amino acids after being mutated. Therefore, for example, Gly75Arg means a modified product in which glycine that is an amino acid at position 75 was mutated to arginine.

**[Table 6]**

| Amino acid substitution | glucose | maltose | lactose | Galactose |
|---|---|---|---|---|
| Wild type | 100 | 90 | 75 | 34 |
| Gly75Trp | 100 | 78 | 88 | 42 |
| Gln168Ser | 100 | 35 | 64 | 16 |
| Gln168Gly | 100 | 24 | 63 | 12 |
| Gln168Tyr | 100 | 74 | 69 | 23 |
| Leu169Phe | 100 | 48 | 78 | 33 |
| Gly295Cys | 100 | 80 | 74 | 36 |
| Gly295Asp | 100 | 64 | 77 | 36 |
| Gly295Glu | 100 | 75 | 72 | 37 |
| Gly295Phe | 100 | 75 | 70 | 34 |
| Gly295Val | 100 | 63 | 125 | 37 |
| Gly295Tyr | 100 | 32 | 81 | 28 |
| Met342Pro | 100 | 78 | 61 | 39 |
| Trp347His | 100 | 86 | 90 | 57 |
| Ala351Thr | 100 | 60 | 86 | 21 |

### [Example 9] Introduction of multiple mutation using mutation scrambling method and consideration of substrate specificity of multiple substitution.

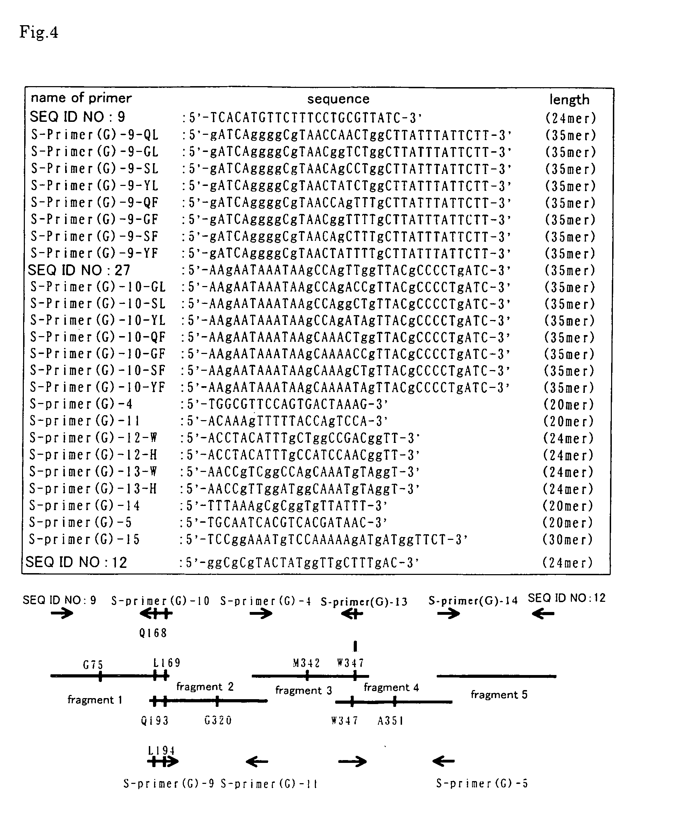

A library of multiple-substituted products having any possible combinations of amino acid substitutions effective in improving the substrate specificitywhichwas apparent from Example 8 was prepared by a mutation scrambling method, thereby attempting to obtain a modified product having higher substrate specificity as compared with the case where only one amino acid substitution is contained. The multiple substitution library was prepared by a biased mutation scrambling method which had been already reported was employed (see the 39^{th} Annual Meeting of Biophysical Society of Japan held at October 6, 2001; Subject No: 1P057; Novartis Pharma K.K.; Biopolymer, Vol. 64 (2002), P.95-105). The biased mutation scrambling method is a modified method of the mutation scrambling method and has a feature capable of freely setting the introduction rate of amino acid substitutions to be combined. In this Example, the rate was set under conditions where G75, G295, M342 and A351 which were located in the vicinity of the substrate binding sites by estimation from a structure model and particularly had high contribution to the substitution specificity included mutations at high rate of 80%; and Q168, L169 and W347 which were thought to have structural features include mutations at the rate of 50%. Specifically, PQQGDH gene was reconstructed by a mutation scrambling method so that the full length of the PQQGDH was divided into 5 fragments shown in Fig. 4 including 1 to 3 amino acid substitutions which were overlapped at the terminal portions and identified in Example 8 were introduced at the above-mentioned rate.

In the mutation scrambling method, firstly, a PCR was carried out using a wild-type gene as a template so as to obtain a partial amplified product (see Fig. 4). The compositions of the reaction solution used in the PCR are shown in Table 7. As the primer, a primer sandwiching G75 and including a sequence corresponding to Q168 and L169 was used.

**[Table 7]**

| |
|---|
| 10×buffer for AccuTaq (SIGMA CORPORATION): 2 µL |
| 10 mM dNTP: 1 µL |
| Forward primer (10 pmol/µL): 1.2 µL |
| Reverse primer (10pmol/µL): 1.2 µL |
| Template (10ng/pL): 0.5 µL |
| dH₂O: 13.9 µL |
| AccuTaq (5units/µL): 0.2 µL |
| Total: 20 µL |

Forward primer: 5'-TCACATGTTCTTTCCTGCGTTATC-3' (SEQ ID NO: 9)

S-primer (G)-10-QL: 5'-AAgAATAAATAAgCCAgTTGgTTACgCCCCTgATC-3'

(SEQ ID NO:27)

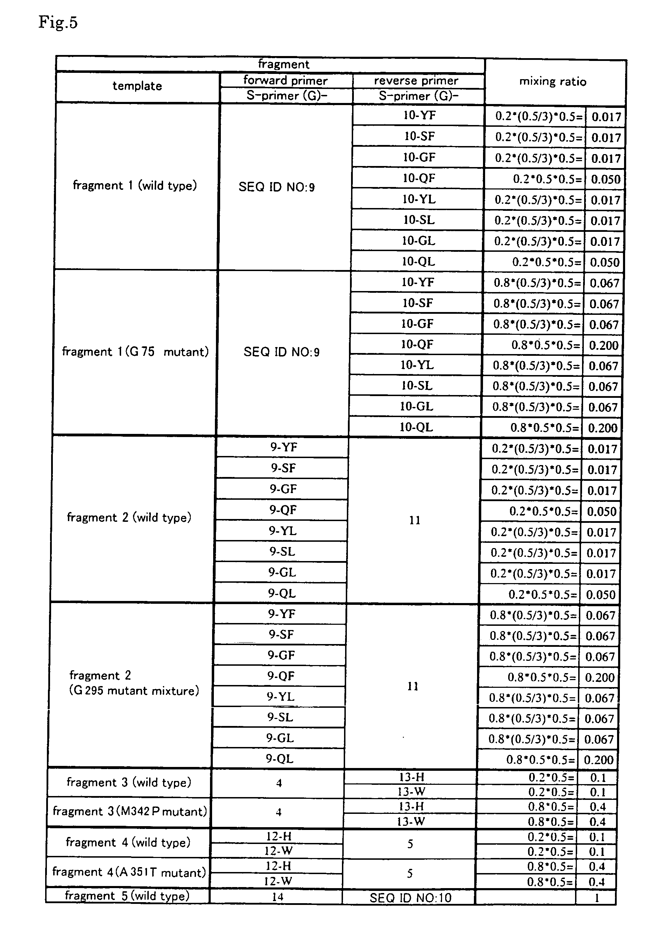

The reaction was carried out under conditions of 98° C for 30 seconds, 30 cycles of 94° C for 5 seconds, 60° C for 20 seconds, and 68° C for 90 seconds, and finally 68° C for 10 minutes. Then, under the same condition, a PCR was carried out by using a modified gene having a mutation at G75 as a template so as to obtain a partial amplified product of the same region as mentioned above. Herein, as shown in Table 6, the number of combinations of the amino acid substitutions at sites G75, Q168 and L169 is 16. The partial amplified products corresponding to all these combinations were prepared by carrying out a reaction using 8 kinds of primers (S-primer(G)-10-QL to S-primer(G)-10-YF) shown in Fig. 4 and using two kinds of genes of wild-type gene and modified type gene as templates. The resultant partial amplified products were mixed at the mixing ratio shown in Fig. 5 so as to obtain a fragment 1 (see fragment 1 in Fig. 4). Fragments 2 to 5 (see fragment 2, fragment 3, fragment 4 and fragment 5 in Fig. 4) were prepared by mixing gene amplified products obtained by using a primer shown in Fig. 4 in the same manner for fragment 1 at the ratio shown in Fig. 5 respectively.

Then, equal amount of each fragment was mixed and a PCR was carried out in a solution having the composition shown in Table 8. The reaction was carried out under conditions of 98° C for 30 seconds, and 5 cycles of 94° C for 5 seconds, 60° C for 20 seconds, and 68° C for 2 minutes. After the PCR was completed, 2.4 µL each of the above-mentioned primer (10 pmol/µL) (SEQ ID NOs: 7 and 8) was added and a PCR was carried out again. At this time, the reaction was carried out under conditions of 94° C for 5 seconds, 25 cycles of 60°C for 20 seconds and 68°C for 2 minutes, and finally 72°C for 10 minutes.

**[Table 8]**

| |
|---|
| mixing fragments: X µL |
| 10×buffer for AccuTaq (SIGMA CORPORATION): 2 µL |
| 10mM dNTP: 1 µL |
| dH₂O: (14.4-X) µL |
| AccuTaq (5 units/pL): 0.2 µL |
| Total: 17.6 µL |

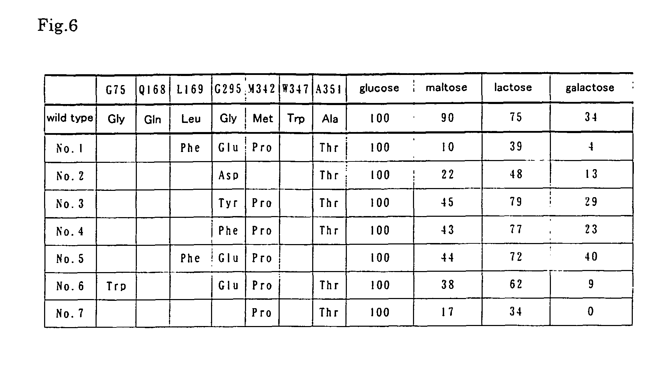

The library of the modified gene obtained by the above-mentioned operation was cut by EcoRI and PstI and substituted by a wild-type gene of pUK-GDHB(S), and then transformation of Escherichia coli was performed. Colony of the transformants were cultured in the method shown in Example 8, then screening was carried out with the PQQGDH activity and the substrate specificity as indices. For clones showing the improvement in the substrate specificity, the base sequence of the modified PQQGDH held by the clone was determined. As a result, as shown in Table 6, it was found that a plurality of modified PQQGDHs having different kinds of combinations of amino acids to be substituted. Note here that in Table of Fig. 6, a mark such as G75 (GGA) means that the number specifies the position of an amino acid and one letter of alphabet at the left of the number represents an amino acid at the position. Furthermore, alphabet in parenthesis means the base sequence encoding the amino acid. On the other hand, a mark such as Phe (TTT) shows substituted product in which the mark (for example, L194 (CTG)) that is located at the top part of the column to which the Phe (TTT) belongs is substituted by an amino acid specified by three letters of alphabet. Furthermore, letters in parenthesis means the base sequence encoding the amino acid.

After each transformant having these modified PQQGDH was purified by the same method as in Example 7, the reactivity with respect to substrate was measured. The measurement results are shown in the right column of Fig. 6. As is apparent from this Table, in any of the obtained modified PQQGDHs, the reactivity with respect to maltose was significantly lowered as compared with that of the wild type PQQGDH. Furthermore, as to the reactivity with respect to lactose, the reactivity was found to be lowered similarly. Furthermore, the reactivity with respect to galactose was greatly lowered in any of the modified PQQGDHs, and in some of the modified PQQGDH, the reactivity was extremely lowered. In this way, we successfully obtained modified PQQGDH having the significantly lowered reactivity with respect to maltose, lactose and galactose, that is, having an extremely excellent substrate specificity. As a result, by combining the mutations at the positions of the amino acid shown in Table of Fig. 6, it was found that the modified PQQGDH having more excellent substrate specificity could be prepared as compared with the case where the mutation is a single.

Herein, the amino acid sequence of No. 1 modified product is shown in SEQ ID NO: 28; the amino acid sequence of No. 2 modified product is shown in SEQ ID NO: 29; the amino acid sequence of No. 3 modified product is shown in SEQ ID NO: 30; the amino acid sequence of No. 4 modified product is shown in SEQ ID NO: 31; the amino acid sequence of No. 5 modified product is shown in SEQ ID NO: 32; the amino acid sequence of No. 6 modified product was shown in SEQ ID NO: 33; and the amino acid sequence of No. 7 modified product was shown in SEQ ID NO: 34, respectively.

In particular, in the No. 1 modified product, the reactivity with respect to maltose is lowered to about 1/9 of the wild-type, and similarly the reactivity with respect to lactose is lowered to about 1/2, and the reactivity with respect to galactose is lowered about 1/9, respectively. Furthermore, also in the No. 6 modified product, the reactivity with respect to maltose is lowered to about 1/2 of the wild-type, and at the same time, the reactivity with respect to lactose is also lowered, and the reactivity with respect to galactose is lowered about 1/4, respectively. Similarly, also in the No. 7 modified product, the reactivity with respect to maltose is lowered to about 1/5 of the wild-type, and at the same time, the reactivity with respect to lactose is also lowered to about 1/2. Substantial reactivity is not shown with respect to galactose. From the comparison of the amino acid substitution sites of the No. 1, No. 6 and No. 7 modified products, it is suggested that the substitution of the amino acid at position 342 and the amino acid at position 351 are particularly important for improvement of the substrate specificity. In particular, it is expected that when amino acid are substituted in both positions, high substrate specificity can be obtained. Furthermore, also the substitution of the amino acid at position 295 is also important, and further, the substitution of the amino acid at position 169 is greatly involved in the improvement of the substrate specificity.

The present invention is not limited only to the description of the above embodiments. A variety of modifications which are within the scopes of the following claims and which are achieved easily by a person skilled in the art are included in the present invention.

### INDUSTRIAL APPLICABILITY

The modified pyrroloquinoline quinone-dependent glucose dehydrogenase provided in the present invention has high specificity with respect to glucose. Therefore, by using such modified enzyme, an amount of glucose can be determined with high accuracy.

## Claims

1. A modified pyrroloquinoline quinone-dependent glucose dehydrogenase, comprising an amino acid sequence in which one or more amino acids in a region corresponding to a first region consisting of amino acids at positions 326 to 354 in pyrroloquinoline quinone-dependent glucose dehydrogenase derived from Acinetobacter calcoaceticus are substituted as compared with an amino acid sequence of the corresponding wild-type enzyme.

2. The modified pyrroloquinoline quinone-dependent glucose dehydrogenase according to claim 1, wherein in said region corresponding to said first region, two or more amino acids including amino acids corresponding to amino acids at positions 342 and 351 are substituted.

3. A modified pyrroloquinoline quinone-dependent glucose dehydrogenase, comprising an amino acid sequence in which in a first region consisting of amino acids at positions 326 to 354 in the amino acid sequence of SEQ ID NO: 1, one or more amino acids are substituted by other amino acids.

4. The modified pyrroloquinoline quinone-dependent glucose dehydrogenase according to claim 3, wherein in said first region, two or more amino acids including amino acids at positions 342 and 351 are substituted by other amino acids.

5. A modified pyrroloquinoline quinone-dependent glucose dehydrogenase, comprising an amino acid sequence in which an amino acid corresponding to an amino acid at position 295 in pyrroloquinoline quinone-dependent glucose dehydrogenase derived from Acinetobacter calcoaceticus is substituted by another amino acid as compared with an amino acid sequence of the corresponding wild-type enzyme.

6. A modified pyrroloquinoline quinone-dependent glucose dehydrogenase, comprising an amino acid sequence in which amino acids corresponding to amino acids of the following (1) and (2) in pyrroloquinoline quinone-dependent glucose dehydrogenase derived from Acinetobacter calcoaceticus are respectively substituted by other amino acids as compared with an amino acid sequence of the corresponding wild-type enzyme:
(1) an amino acid at position 295; and
(2) one or more amino acids selected from the group consisting of amino acids at positions 75, 168, 169, 342, 347 and 351.

7. A modified pyrroloquinoline quinone-dependent glucose dehydrogenase, comprisinganyof the following amino acid sequences (1) to (6):
(1) an amino acid sequence in which amino acids corresponding to amino acids at positions 169, 295, 342 and 351 in pyrroloquinoline quinone-dependent glucose dehydrogenase derived from Acinetobacter calcoaceticus are respectively substituted by other amino acids, as compared with an amino acid sequence of the corresponding wild-type enzyme;
(2) an amino acid sequence in which amino acids corresponding to amino acids at positions 295 and 351 in pyrroloquinoline quinone-dependent glucose dehydrogenase derived from Acinetobacter calcoaceticus are respectively substituted by other amino acids, as compared with an amino acid sequence of the corresponding wild-type enzyme;
(3) an amino acid sequence in which amino acids corresponding to amino acids at positions 295, 342 and 351 in pyrroloquinoline quinone-dependent glucose dehydrogenase derived from Acinetobacter calcoaceticus are respectively substituted by other amino acids, as compared with an amino acid sequence of the corresponding wild-type enzyme;
(4) an amino acid sequence in which amino acids corresponding to amino acids at positions 169, 295 and 342 in pyrroloquinoline quinone-dependent glucose dehydrogenase derived from Acinetobacter calcoaceticus are respectively substituted by other amino acids, as compared with an amino acid sequence of the corresponding wild-type enzyme;
(5) an amino acid sequence in which amino acids corresponding to amino acids at positions 75, 295, 342 and 351 in pyrroloquinoline quinone-dependent glucose dehydrogenase derived from Acinetobacter calcoaceticus are respectively substituted by other amino acids, as compared with an amino acid sequence of the corresponding wild-type enzyme; and
(6) an amino acid sequence in which amino acids corresponding to amino acids at positions 342 and 351 in pyrroloquinoline quinone-dependent glucose dehydrogenase derived from Acinetobacter calcoaceticus are respectively substituted by other amino acids, as compared with an amino acid sequence of the corresponding wild-type enzyme.

8. A modified pyrroloquinoline quinone-dependent glucose dehydrogenase,comprising any of thefollowing amino acid sequences (1) to (6):
(1) an amino acid sequence of SEQ ID NO: 1 in which amino acids at positions 169, 295, 342 and 351 are substituted by other amino acids respectively;
(2) an amino acid sequence of SEQ ID NO: 1 in which amino acids at positions 295 and 351 are substituted by other amino acids respectively;
(3) an amino acid sequence of SEQ ID NO: 1 in which amino acids at positions 295, 342, and 351 are substituted by other amino acids respectively;
(4) an amino acid sequence of SEQ ID NO: 1 in which amino acids at positions 169, 295 and 342 are substituted by other amino acids respectively;
(5) an amino acid sequence of SEQ ID NO: 1 in which amino acids at positions 75, 295, 342 and 351 are substituted by other amino acids respectively; and
(6) an amino acid sequence of SEQ ID NO: 1 in which amino acids at positions 342 and 351 are substituted by other amino acids respectively.

9. A modified pyrroloquinoline quinone-dependent glucose dehydrogenase, comprising any of amino acid sequences of SEQ ID NOs: 28 to 34.

10. A modified pyrroloquinoline quinone-dependent glucose dehydrogenase, wherein the reactivity with respect to maltose is 40% or less and the reactivity with respect to galactose is 10% or less, respectively relative to the reactivity with respect to glucose.

11. A modified pyrroloquinoline quinone-dependent glucose dehydrogenase, wherein the reactivity with respect to maltose is 20% or less and the reactivity with respect to galactose is 5% or less, respectively relative to the reactivity with respect to glucose.

12. A modified pyrroloquinoline quinone-dependent glucose dehydrogenase, wherein the reactivity with respect to galactose is 10% or less relative to the reactivity with respect to glucose.

13. An isolated polynucleotide encoding the modified pyrroloquinoline quinone-dependent glucose dehydrogenase described in any of claims 1 to 12.

14. A vector containing polynucleotide described in claim 13.

15. A transformant holding polynucleotide described in claim 13.

16. A method for producing pyrroloquinoline quinone-dependent glucose dehydrogenase, the method comprising
a step of culturing the transformant described in claim 15 in a state in which the polynucleotide can be expressed; and
a step of separating the expressed product of the polynucleotide.

17. A kit for measuring glucose, comprising the modified pyrroloquinoline quinone-dependent glucose dehydrogenase described in any of claims 1 to 12.


SEQUENCE LISTING

<110> AMANO ENZYME INC.

<120> PYRROLOQUINOLINE QUINONE-DEPENDENT GLUCOSE DEHYDROGENASE

<130> P0204202

<160> 34

<170> PatentIn version 3.1

<210> 1
<211> 455
<212> PRT
<213> Acinetobacter calcoaceticus

<400> 1

Asp Val Pro Leu Thr Pro Ser Gln Phe Ala Lys Ala Lys Thr Glu Ser
1               5                   10                  15

Phe Asp Lys Lys Val Leu Leu Ser Asn Leu Asn Lys Pro His Ala Leu

            20                  25                  30

Leu Trp Gly Pro Asp Asn Gln Ile Trp Leu Thr Glu Arg Ala Thr Gly
        35                  40                  45

Lys Ile Leu Arg Val Asn Pro Glu Ser Gly Ser Val Lys Thr Val Phe
    50                  55                  60

Gln Val Pro Glu Ile Val Asn Asp Ala Asp Gly Gln Asn Gly Leu Leu
65                  70                  75                  80

Gly Phe Ala Phe His Pro Asp Phe Lys Asn Asn Pro Tyr Ile Tyr Val
                85                  90                  95

Ser Gly Thr Phe Lys Asn Pro Lys Ser Thr Asp Lys Glu Leu Pro Asn
            100                 105                 110

Gln Thr Ile Ile Arg Arg Tyr Thr Tyr Asn Lys Ala Thr Asp Thr Leu
        115                 120                 125

Glu Lys Pro Val Asp Leu Leu Ala Gly Leu Pro Ser Ser Lys Asp His
    130                 135                 140

Gln Ser Gly Arg Leu Val Ile Gly Pro Asp Gln Lys Ile Tyr Tyr Thr
145                 150                 155                 160

Ile Gly Asp Gln Gly Arg Asn Gln Leu Ala Tyr Leu Phe Leu Pro Asn
                165                 170                 175

Gln Ala Gln His Thr Pro Thr Gln Gln Glu Leu Ser Gly Lys Asp Tyr
            180                 185                 190

His Thr Tyr Met Gly Lys Val Leu Arg Leu Asn Leu Asp Gly Ser Ile
        195                 200                 205

Pro Lys Asp Asn Pro Ser Phe Asn Gly Val Ile Ser His Ile Tyr Thr
    210                 215                 220

Leu Gly His Arg Asn Pro Gln Gly Leu Ala Phe Thr Pro Asn Gly Lys
225                 230                 235                 240

Leu Leu Gln Ser Glu Gln Gly Pro Asn Ser Asp Asp Glu Ile Asn Leu
                245                 250                 255

Ile Val Lys Gly Gly Asn Tyr Gly Trp Pro Asn Val Ala Gly Tyr Lys
            260                 265                 270

Asp Asp Ser Gly Tyr Ala Tyr Ala Asn Tyr Ser Ala Ala Ser Asn Lys
        275                 280                 285

Ala Gln Ile Lys Asp Leu Gly Gln Asn Gly Leu Lys Val Ala Ala Gly
    290                 295                 300

Val Pro Val Thr Lys Glu Ser Glu Trp Thr Gly Lys Asn Phe Val Pro
305                 310                 315                 320

Pro Leu Lys Thr Leu Tyr Thr Val Gln Asp Thr Tyr Asn Tyr Asn Asp
                325                 330                 335
Pro Thr Cys Gly Asp Met Thr Tyr Ile Cys Trp Pro Thr Val Ala Pro
            340                 345                 350
Ser Ser Ala Tyr Val Tyr Lys Gly Gly Lys Lys Ala Ile Ser Gly Trp
        355                 360                 365
Glu Asn Thr Leu Leu Val Pro Ser Leu Lys Arg Gly Val Ile Phe Arg
    370                 375                 380
Ile Lys Leu Asp Pro Thr Tyr Ser Ala Thr Tyr Asp Asp Ala Val Pro
385                 390                 395                 400
Met Phe Lys Ser Asn Asn Arg Tyr Arg Asp Val Ile Ala Ser Pro Asp
                405                 410                 415
Gly Asn Val Leu Tyr Val Leu Thr Asp Thr Ser Gly Asn Val Gln Lys

            420                 425                 430

Asp Asp Gly Ser Val Thr Asn Thr Leu Glu Asn Pro Gly Ser Leu Ile

        435                 440                 445

Lys Phe Thr Tyr Lys Ala Lys

    450                 455

<210> 2

<211> 1368

<212> DNA

<213> Acinetobacter calcoaceticus

<400> 2

gatgttcctc ttacaccatc tcaatttgct aaagcgaaaa cagaaagctt tgacaagaaa 60

gttcttctat ctaatttaaa taagccacat gctttgttgt gggggcctga taatcaaatt 120

tggttaacgg agcgggcaac agggaagatt ctaagagtga atccagagtc gggcagtgta 180

aaaacagttt ttcaggttcc tgagattgta aatgatgctg atggacaaaa cggtttattg 240

ggttttgcct ttcatcctga ctttaaaaat aatccttata tctatgtttc aggtactttt 300

aaaaatccga aatctacaga taaagaatta ccgaatcaaa ctattattcg tcgatatacc 360

tataacaaag caacagatac tcttgagaaa ccagtagatt tattagcagg attaccttca 420

tcgaaagacc atcagtcggg tcgccttgtc attggtccag accaaaagat ttactatacg 480

attggtgatc aggggcgtaa ccagctggct tatttattct taccaaatca agcacagcat 540

acgccgactc aacaggaact gagcggcaaa gactatcata cctatatggg taaagtatta 600

cgcttaaatc tggatggaag tattccaaaa gataatccaa gctttaacgg tgtaattagc 660

catatttata cgctcggtca tcgtaatcca cagggcttgg catttactcc aaatggtaaa 720

ctgttgcaat ctgaacaggg tccaaactct gacgatgaaa ttaacctcat tgtcaaaggt 780

ggtaactatg gctggccaaa tgtagcgggt tataaagatg atagtggtta tgcctatgca 840

aattattcgg cagcaagcaa taaagcacaa attaaagatt taggacaaaa tggtttaaaa 900

gtggcagctg gcgttccagt gactaaagag tctgaatgga ctggtaaaaa ctttgtaccg 960

ccgttaaaaa ctttatatac cgtccaagat acctataact ataatgaccc aacctgtggg 1020

gatatgacct acatttgctg gccaacggtt gcgccgtcat ctgcttatgt ctataaggga 1080

ggcaaaaaag caatttctgg ttgggaaaat accttattgg ttccatcttt aaagcgcggt 1140

gttattttcc gtattaagct agatccaact tacagtgcta cttatgatga tgctgtgccg 1200

atgtttaaga gcaacaatcg ttatcgtgac gtgattgcaa gtccagatgg aaatgtttta 1260

tatgtattga ctgatacttc cggaaatgtc caaaaagatg atggttctgt aacgaataca 1320

ttagaaaacc caggatctct cattaagttc acctataagg ctaagtaa 1368

<210> 3

<211> 480

<212> PRT

<213> Acinetobacter calcoaceticus

<400> 3

Met Asn Lys His Leu Leu Ala Lys Ile Thr Leu Leu Gly Ala Ala Gln

1               5                   10                  15

Leu Leu Thr Leu Asn Ser Ala Phe Ala Asp Val Pro Leu Thr Pro Ser
            20                  25                  30
Gln Phe Ala Lys Ala Lys Thr Glu Ser Phe Asp Lys Lys Val Leu Leu
        35                  40                  45
Ser Asn Leu Asn Lys Pro His Ala Leu Leu Trp Gly Pro Asp Asn Gln
    50                  55                  60
Ile Trp Leu Thr Glu Arg Ala Thr Gly Lys Ile Leu Arg Val Asn Pro
65                  70                  75                  80
Glu Ser Gly Ser Val Lys Thr Val Phe Gln Val Pro Glu Ile Val Asn
                85                  90                  95
Asp Ala Asp Gly Gln Asn Gly Leu Leu Gly Phe Ala Phe His Pro Asp
            100                 105                 110
Phe Lys Asn Asn Pro Tyr Ile Tyr Val Ser Gly Thr Phe Lys Asn Pro

        115                 120                 125

Lys Ser Thr Asp Lys Glu Leu Pro Asn Gln Thr Ile Ile Arg Arg Tyr
    130                 135                 140

Thr Tyr Asn Lys Ala Thr Asp Thr Leu Glu Lys Pro Val Asp Leu Leu
145                 150                 155                 160

Ala Gly Leu Pro Ser Ser Lys Asp His Gln Ser Gly Arg Leu Val Ile
                165                 170                 175

Gly Pro Asp Gln Lys Ile Tyr Tyr Thr Ile Gly Asp Gln Gly Arg Asn
            180                 185                 190

Gln Leu Ala Tyr Leu Phe Leu Pro Asn Gln Ala Gln His Thr Pro Thr
        195                 200                 205

Gln Gln Glu Leu Ser Gly Lys Asp Tyr His Thr Tyr Met Gly Lys Val
    210                 215                 220

Leu Arg Leu Asn Leu Asp Gly Ser Ile Pro Lys Asp Asn Pro Ser Phe
225                 230                 235                 240

Asn Gly Val Ile Ser His Ile Tyr Thr Leu Gly His Arg Asn Pro Gln
                245                 250                 255

Gly Leu Ala Phe Thr Pro Asn Gly Lys Leu Leu Gln Ser Glu Gln Gly
            260                 265                 270

Pro Asn Ser Asp Asp Glu Ile Asn Leu Ile Val Lys Gly Gly Asn Tyr
        275                 280                 285

Gly Trp Pro Asn Val Ala Gly Tyr Lys Asp Asp Ser Gly Tyr Ala Tyr
    290                 295                 300

Ala Asn Tyr Ser Ala Ala Ser Asn Lys Ala Gln Ile Lys Asp Leu Gly
305                 310                 315                 320

Gln Asn Gly Leu Lys Val Ala Ala Gly Val Pro Val Thr Lys Glu Ser
                325                 330                 335
Glu Trp Thr Gly Lys Asn Phe Val Pro Pro Leu Lys Thr Leu Tyr Thr
            340                 345                 350
Val Gln Asp Thr Tyr Asn Tyr Asn Asp Pro Thr Cys Gly Asp Met Thr
        355                 360                 365
Tyr Ile Cys Trp Pro Thr Val Ala Pro Ser Ser Ala Tyr Val Tyr Lys
    370                 375                 380
Gly Gly Lys Lys Ala Ile Ser Gly Trp Glu Asn Thr Leu Leu Val Pro
385                 390                 395                 400
Ser Leu Lys Arg Gly Val Ile Phe Arg Ile Lys Leu Asp Pro Thr Tyr
                405                 410                 415

Ser Ala Thr Tyr Asp Asp Ala Val Pro Met Phe Lys Ser Asn Asn Arg

            420                 425                 430

Tyr Arg Asp Val Ile Ala Ser Pro Asp Gly Asn Val Leu Tyr Val Leu

        435                 440                 445

Thr Asp Thr Ser Gly Asn Val Gln Lys Asp Asp Gly Ser Val Thr Asn

    450                 455                 460

Thr Leu Glu Asn Pro Gly Ser Leu Ile Lys Phe Thr Tyr Lys Ala Lys

465                 470                 475                 480

<210> 4

<211> 1443

<212> DNA

<213> Acinetobacter calcoaceticus

<400> 4

atgaataaac atttattggc taaaattact ttattaggcg ctgctcagct acttacgctc 60

aattcagcat ttgctgatgt tcctcttaca ccatctcaat ttgctaaagc gaaaacagaa 120

agctttgaca agaaagttct tctatctaat ttaaataagc cacatgcttt gttgtggggg 180

cctgataatc aaatttggtt aacggagcgg gcaacaggga agattctaag agtgaatcca 240

gagtcgggca gtgtaaaaac agtttttcag gttcctgaga ttgtaaatga tgctgatgga 300

caaaacggtt tattgggttt tgcctttcat cctgacttta aaaataatcc ttatatctat 360

gtttcaggta cttttaaaaa tccgaaatct acagataaag aattaccgaa tcaaactatt 420

attcgtcgat atacctataa caaagcaaca gatactcttg agaaaccagt agatttatta 480

gcaggattac cttcatcgaa agaccatcag tcgggtcgcc ttgtcattgg tccagaccaa 540

aagatttact atacgattgg tgatcagggg cgtaaccagc tggcttattt attcttacca 600

aatcaagcac agcatacgcc gactcaacag gaactgagcg gcaaagacta tcatacctat 660

atgggtaaag tattacgctt aaatctggat ggaagtattc caaaagataa tccaagcttt 720

aacggtgtaa ttagccatat ttatacgctc ggtcatcgta atccacaggg cttggcattt 780

actccaaatg gtaaactgtt gcaatctgaa cagggtccaa actctgacga tgaaattaac 840

ctcattgtca aaggtggtaa ctatggctgg ccaaatgtag cgggttataa agatgatagt 900

ggttatgcct atgcaaatta ttcggcagca agcaataaag cacaaattaa agatttagga 960

caaaatggtt taaaagtggc agctggcgtt ccagtgacta aagagtctga atggactggt 1020

aaaaactttg taccgccgtt aaaaacttta tataccgtcc aagataccta taactataat 1080

gacccaacct gtggggatat gacctacatt tgctggccaa cggttgcgcc gtcatctgct 1140

tatgtctata agggaggcaa aaaagcaatt tctggttggg aaaatacctt attggttcca 1200

tctttaaagc gcggtgttat tttccgtatt aagctagatc caacttacag tgctacttat 1260

gatgatgctg tgccgatgtt taagagcaac aatcgttatc gtgacgtgat tgcaagtcca 1320

gatggaaatg ttttatatgt attgactgat acttccggaa atgtccaaaa agatgatggt 1380

tctgtaacga atacattaga aaacccagga tctctcatta agttcaccta taaggctaag 1440

taa 1443

<210> 5

<211> 21

<212> DNA
<213> Artificial Sequence

<220>
<223> Description of Artificial Sequence : sense primer

<400> 5
acaaatcata tagagaactc g 21

<210> 6
<211> 40
<212> DNA
<213> Artificial Sequence

<220>
<223> Description of Artificial Sequence : antisense primer

<400> 6
ttacttagcc ttataggtga acttaatgag agatcctggg 40

<210> 7
<211> 48
<212> DNA

<213> Artificial Sequence

<220>
<223> Description of Artificial Sequence : sense primer

<400> 7
gcggccgcga attcatgaat aaacatttat tggctaaaat tactttat 48

<210> 8
<211> 57
<212> DNA
<213> Artificial Sequence

<220>
<223> Description of Artificial Sequence : antisense primer

<400> 8
gcggccgcct gcagctatta cttagcctta taggtgaact taatgagaga tcctggg 57

<210> 9
<211> 24
<212> DNA
<213> Artificial Sequence

<220>

<223> Description of Artificial Sequence : sense primer

<400> 9

tcacatgttc tttcctgcgt tatc 24

<210> 10

<211> 46

<212> DNA

<213> Artificial Sequence

<220>

<223> Description of Artificial Sequence : antisense primer

<400> 10

atggtgatgg tgatggtgct tagccttata ggtgaactta atgaga 46

<210> 11

<211> 42

<212> DNA

<213> Artificial Sequence

<220>

<223> Description of Artificial Sequence : antisense primer

<400> 11
gcggccgcct gcagctatta atggtgatgg tgcttagcct ta 42

<210> 12
<211> 23
<212> DNA
<213> Artificial Sequence

<220>
<223> Description of Artificial Sequence : antisense primer

<400> 12
ggcgcgtact atggttgctt tga 23

<210> 13
<211> 35
<212> DNA
<213> Artificial Sequence

<220>

<223> Description of Artificial Sequence : sense primer GDHB/G75-F

<220>
<221> misc_feature
<222> (16)..(18)
<223> n stands for any base

<400> 13
gtaaatgatg ctgatnnnca aaacggttta ttggg 35

<210> 14
<211> 35
<212> DNA
<213> Artificial Sequence

<220>
<223> Description of Artificial Sequence : antisense primer GDHB/G75-R

<220>
<221> misc_feature
<222> (18)..(20)
<223> n stands for any base

<400> 14
cccaataaac cgttttgnnn atcagcatca tttac 35

<210> 15
<211> 38
<212> DNA
<213> Artificial Sequence

<220>
<223> Description of Artificial Sequence : sense primer GDHB/G295-F

<220>
<221> misc_feature
<222> (16)..(18)
<223> n stands for any base

<400> 15
caaattaaag atttannnca aaatggttta aaagtggc 38

<210> 16
<211> 38

<212> DNA
<213> Artificial Sequence

<220>
<223> Description of Artificial Sequence : antisense primer GDHB/G295-R

<220>
<221> misc_feature
<222> (21)..(23)
<223> n stands for any base

<400> 16
gccactttta aaccattttg nnntaaatct ttaatttg 38

<210> 17
<211> 33
<212> DNA
<213> Artificial Sequence

<220>
<223> Description of Artificial Sequence : sense primer GDHB/M342-F

<220>

<221>  misc_feature
<222>  (16)..(18)
<223>  n stands for any base

<400>  17
ccaacctgtg gggatnnnac ctacatttgc tgg                                    33

<210>  18
<211>  33
<212>  DNA
<213>  Artificial Sequence

<220>
<223>  Description of Artificial Sequence : antisense primer GDHB/M342-R

<220>
<221>  misc_feature
<222>  (16)..(18)
<223>  n stands for any base

<400>  18
ccagcaaatg taggtnnnat ccccacaggt tgg                                    33

<210> 19

<211> 33

<212> DNA

<213> Artificial Sequence

<220>

<223> Description of Artificial Sequence : sense primer GDHB/A351-F

<220>

<221> misc_feature

<222> (11)..(13)

<223> n stands for any base

<400> 19

gccaacggtt nnnccgtcat ctgcttatgt cta 33

<210> 20

<211> 33

<212> DNA

<213> Artificial Sequence

<220>

<223> Description of Artificial Sequence : sense primer GDHB/A351-R

<220>

<221> misc_feature

<222> (21)..(23)

<223> n stands for any base

<400> 20

tagacataag cagatgacgg nnnaaccgtt ggc 33

<210> 21

<211> 33

<212> DNA

<213> Artificial Sequence

<220>

<223> Description of Artificial Sequence : antisense primer GDHB/Q168-F

<220>

<221> misc_feature

<222> (16)..(18)

<223> n stands for any base

<400> 21
gatcaggggc gtaacnnnct ggcttattta ttc                                    33

<210> 22
<211> 33
<212> DNA
<213> Artificial Sequence

<220>
<223> Description of Artificial Sequence : sense primer GDHB/Q168-F

<220>
<221> misc_feature
<222> (16)..(18)
<223> n stands for any base

<400> 22
gaataaataa gccagnnngt tacgcccctg atc                                    33

<210> 23

<211> 33

<212> DNA

<213> Artificial Sequence

<220>

<223> Description of Artificial Sequence : sense primer GDHB/L169-F

<220>

<221> misc_feature

<222> (14)..(16)

<223> n stands for any base

<400> 23

ggggcgtaac cagnnngctt atttattctt acc 33

<210> 24

<211> 33

<212> DNA

<213> Artificial Sequence

<220>

<223> Description of Artificial Sequence : antisense primer GDHB/L169-R

<220>

<221> misc_feature

<222> (18)..(20)

<223> n stands for any base

<400> 24

ggtaagaata aataagcnnn ctggttacgc ccc 33

<210> 25

<211> 37

<212> DNA

<213> Artificial Sequence

<220>

<223> Description of Artificial Sequence : sense primer GDHB/W347-F

<220>

<221> misc_feature

<222> (20)..(22)

<223> n stands for any base

<400> 25

ggatatgacc tacatttgcn nnccaacggt tgcgccg 37

<210> 26
<211> 37
<212> DNA
<213> Artificial Sequence

<220>
<223> Description of Artificial Sequence : antisense primer GDHB/W347-R

<220>
<221> misc_feature
<222> (16)..(18)
<223> n stands for any base

<400> 26
cggcgcaacc gttggnnngc aaatgtaggt catatcc 37

<210> 27
<211> 35
<212> DNA
<213> Artificial Sequence

<220>

<223> Description of Artificial Sequence : antisense primer

<400> 27
aagaataaat aagccagttg gttacgcccc tgatc 35

<210> 28
<211> 455
<212> PRT
<213> Acinetobacter calcoaceticus

<220>
<221> MUTAGEN
<222> (169)..(169)
<223>

<220>
<221> MUTAGEN
<222> (295)..(295)
<223>

<220>
<221> MUTAGEN

<222>  (342)..(342)

<223>

<220>

<221>  MUTAGEN

<222>  (351)..(351)

<223>

<400>  28

Asp Val Pro Leu Thr Pro Ser Gln Phe Ala Lys Ala Lys Thr Glu Ser

1               5                   10                  15

Phe Asp Lys Lys Val Leu Leu Ser Asn Leu Asn Lys Pro His Ala Leu

            20                  25                  30

Leu Trp Gly Pro Asp Asn Gln Ile Trp Leu Thr Glu Arg Ala Thr Gly

        35                  40                  45

Lys Ile Leu Arg Val Asn Pro Glu Ser Gly Ser Val Lys Thr Val Phe

    50                  55                  60

Gln Val Pro Glu Ile Val Asn Asp Ala Asp Gly Gln Asn Gly Leu Leu
65                  70                  75                  80
Gly Phe Ala Phe His Pro Asp Phe Lys Asn Asn Pro Tyr Ile Tyr Val
                85                  90                  95
Ser Gly Thr Phe Lys Asn Pro Lys Ser Thr Asp Lys Glu Leu Pro Asn
            100                 105                 110
Gln Thr Ile Ile Arg Arg Tyr Thr Tyr Asn Lys Ala Thr Asp Thr Leu
        115                 120                 125
Glu Lys Pro Val Asp Leu Leu Ala Gly Leu Pro Ser Ser Lys Asp His
    130                 135                 140
Gln Ser Gly Arg Leu Val Ile Gly Pro Asp Gln Lys Ile Tyr Tyr Thr
145                 150                 155                 160

Ile Gly Asp Gln Gly Arg Asn Gln Phe Ala Tyr Leu Phe Leu Pro Asn
                165                 170                 175
Gln Ala Gln His Thr Pro Thr Gln Gln Glu Leu Ser Gly Lys Asp Tyr
            180                 185                 190
His Thr Tyr Met Gly Lys Val Leu Arg Leu Asn Leu Asp Gly Ser Ile
        195                 200                 205
Pro Lys Asp Asn Pro Ser Phe Asn Gly Val Ile Ser His Ile Tyr Thr
    210                 215                 220
Leu Gly His Arg Asn Pro Gln Gly Leu Ala Phe Thr Pro Asn Gly Lys
225                 230                 235                 240
Leu Leu Gln Ser Glu Gln Gly Pro Asn Ser Asp Asp Glu Ile Asn Leu
                245                 250                 255
Ile Val Lys Gly Gly Asn Tyr Gly Trp Pro Asn Val Ala Gly Tyr Lys

            260                 265                 270

Asp Asp Ser Gly Tyr Ala Tyr Ala Asn Tyr Ser Ala Ala Ser Asn Lys
        275                 280                 285

Ala Gln Ile Lys Asp Leu Glu Gln Asn Gly Leu Lys Val Ala Ala Gly
    290                 295                 300

Val Pro Val Thr Lys Glu Ser Glu Trp Thr Gly Lys Asn Phe Val Pro
305                 310                 315                 320

Pro Leu Lys Thr Leu Tyr Thr Val Gln Asp Thr Tyr Asn Tyr Asn Asp
                325                 330                 335

Pro Thr Cys Gly Asp Pro Thr Tyr Ile Cys Trp Pro Thr Val Thr Pro
            340                 345                 350

Ser Ser Ala Tyr Val Tyr Lys Gly Gly Lys Lys Ala Ile Ser Gly Trp
        355                 360                 365

Glu Asn Thr Leu Leu Val Pro Ser Leu Lys Arg Gly Val Ile Phe Arg
    370                 375                 380

Ile Lys Leu Asp Pro Thr Tyr Ser Ala Thr Tyr Asp Asp Ala Val Pro
385                 390                 395                 400

Met Phe Lys Ser Asn Asn Arg Tyr Arg Asp Val Ile Ala Ser Pro Asp
                405                 410                 415

Gly Asn Val Leu Tyr Val Leu Thr Asp Thr Ser Gly Asn Val Gln Lys
            420                 425                 430

Asp Asp Gly Ser Val Thr Asn Thr Leu Glu Asn Pro Gly Ser Leu Ile
        435                 440                 445

Lys Phe Thr Tyr Lys Ala Lys
    450                 455

<210> 29

<211> 455

<212> PRT

<213> Acinetobacter calcoaceticus

<220>

<221> MUTAGEN

<222> (295)..(295)

<223>

<220>

<221> MUTAGEN

<222> (351)..(351)

<223>

<400> 29

Asp Val Pro Leu Thr Pro Ser Gln Phe Ala Lys Ala Lys Thr Glu Ser
1               5                   10                  15

Phe Asp Lys Lys Val Leu Leu Ser Asn Leu Asn Lys Pro His Ala Leu
            20                  25                  30

Leu Trp Gly Pro Asp Asn Gln Ile Trp Leu Thr Glu Arg Ala Thr Gly
        35                  40                  45
Lys Ile Leu Arg Val Asn Pro Glu Ser Gly Ser Val Lys Thr Val Phe
    50                  55                  60
Gln Val Pro Glu Ile Val Asn Asp Ala Asp Gly Gln Asn Gly Leu Leu
65                  70                  75                  80
Gly Phe Ala Phe His Pro Asp Phe Lys Asn Asn Pro Tyr Ile Tyr Val
                85                  90                  95
Ser Gly Thr Phe Lys Asn Pro Lys Ser Thr Asp Lys Glu Leu Pro Asn
            100                 105                 110
Gln Thr Ile Ile Arg Arg Tyr Thr Tyr Asn Lys Ala Thr Asp Thr Leu
        115                 120                 125

Glu Lys Pro Val Asp Leu Leu Ala Gly Leu Pro Ser Ser Lys Asp His
    130                 135                 140

Gln Ser Gly Arg Leu Val Ile Gly Pro Asp Gln Lys Ile Tyr Tyr Thr
145                 150                 155                 160

Ile Gly Asp Gln Gly Arg Asn Gln Leu Ala Tyr Leu Phe Leu Pro Asn
                165                 170                 175

Gln Ala Gln His Thr Pro Thr Gln Gln Glu Leu Ser Gly Lys Asp Tyr
            180                 185                 190

His Thr Tyr Met Gly Lys Val Leu Arg Leu Asn Leu Asp Gly Ser Ile
        195                 200                 205

Pro Lys Asp Asn Pro Ser Phe Asn Gly Val Ile Ser His Ile Tyr Thr
    210                 215                 220

Leu Gly His Arg Asn Pro Gln Gly Leu Ala Phe Thr Pro Asn Gly Lys

225                 230                 235                 240

Leu Leu Gln Ser Glu Gln Gly Pro Asn Ser Asp Asp Glu Ile Asn Leu
                245                 250                 255

Ile Val Lys Gly Gly Asn Tyr Gly Trp Pro Asn Val Ala Gly Tyr Lys
            260                 265                 270

Asp Asp Ser Gly Tyr Ala Tyr Ala Asn Tyr Ser Ala Ala Ser Asn Lys
        275                 280                 285

Ala Gln Ile Lys Asp Leu Asp Gln Asn Gly Leu Lys Val Ala Ala Gly
    290                 295                 300

Val Pro Val Thr Lys Glu Ser Glu Trp Thr Gly Lys Asn Phe Val Pro
305                 310                 315                 320

Pro Leu Lys Thr Leu Tyr Thr Val Gln Asp Thr Tyr Asn Tyr Asn Asp
                325                 330                 335

Pro Thr Cys Gly Asp Met Thr Tyr Ile Cys Trp Pro Thr Val Thr Pro
            340                 345                 350
Ser Ser Ala Tyr Val Tyr Lys Gly Gly Lys Lys Ala Ile Ser Gly Trp
        355                 360                 365
Glu Asn Thr Leu Leu Val Pro Ser Leu Lys Arg Gly Val Ile Phe Arg
    370                 375                 380
Ile Lys Leu Asp Pro Thr Tyr Ser Ala Thr Tyr Asp Asp Ala Val Pro
385                 390                 395                 400
Met Phe Lys Ser Asn Asn Arg Tyr Arg Asp Val Ile Ala Ser Pro Asp
                405                 410                 415
Gly Asn Val Leu Tyr Val Leu Thr Asp Thr Ser Gly Asn Val Gln Lys
            420                 425                 430

Asp Asp Gly Ser Val Thr Asn Thr Leu Glu Asn Pro Gly Ser Leu Ile

        435                 440                 445

Lys Phe Thr Tyr Lys Ala Lys

    450                 455

<210> 30

<211> 455

<212> PRT

<213> Acinetobacter calcoaceticus

<220>

<221> MUTAGEN

<222> (295)..(295)

<223>

<220>

<221> MUTAGEN

<222> (342)..(342)

<223>

<220>

<221>  MUTAGEN

<222>  (351)..(351)

<223>

<400>  30

Asp Val Pro Leu Thr Pro Ser Gln Phe Ala Lys Ala Lys Thr Glu Ser

1               5                   10                  15

Phe Asp Lys Lys Val Leu Leu Ser Asn Leu Asn Lys Pro His Ala Leu

            20                  25                  30

Leu Trp Gly Pro Asp Asn Gln Ile Trp Leu Thr Glu Arg Ala Thr Gly

        35                  40                  45

Lys Ile Leu Arg Val Asn Pro Glu Ser Gly Ser Val Lys Thr Val Phe

    50                  55                  60

Gln Val Pro Glu Ile Val Asn Asp Ala Asp Gly Gln Asn Gly Leu Leu

65                  70                  75                  80

Gly Phe Ala Phe His Pro Asp Phe Lys Asn Asn Pro Tyr Ile Tyr Val
                85                  90                  95
Ser Gly Thr Phe Lys Asn Pro Lys Ser Thr Asp Lys Glu Leu Pro Asn
            100                 105                 110
Gln Thr Ile Ile Arg Arg Tyr Thr Tyr Asn Lys Ala Thr Asp Thr Leu
        115                 120                 125
Glu Lys Pro Val Asp Leu Leu Ala Gly Leu Pro Ser Ser Lys Asp His
    130                 135                 140
Gln Ser Gly Arg Leu Val Ile Gly Pro Asp Gln Lys Ile Tyr Tyr Thr
145                 150                 155                 160
Ile Gly Asp Gln Gly Arg Asn Gln Leu Ala Tyr Leu Phe Leu Pro Asn
                165                 170                 175

Gln Ala Gln His Thr Pro Thr Gln Gln Glu Leu Ser Gly Lys Asp Tyr
            180                 185                 190
His Thr Tyr Met Gly Lys Val Leu Arg Leu Asn Leu Asp Gly Ser Ile
        195                 200                 205
Pro Lys Asp Asn Pro Ser Phe Asn Gly Val Ile Ser His Ile Tyr Thr
    210                 215                 220
Leu Gly His Arg Asn Pro Gln Gly Leu Ala Phe Thr Pro Asn Gly Lys
225                 230                 235                 240
Leu Leu Gln Ser Glu Gln Gly Pro Asn Ser Asp Asp Glu Ile Asn Leu
                245                 250                 255
Ile Val Lys Gly Gly Asn Tyr Gly Trp Pro Asn Val Ala Gly Tyr Lys
            260                 265                 270
Asp Asp Ser Gly Tyr Ala Tyr Ala Asn Tyr Ser Ala Ala Ser Asn Lys

        275                 280                 285

Ala Gln Ile Lys Asp Leu Tyr Gln Asn Gly Leu Lys Val Ala Ala Gly

    290                 295                 300

Val Pro Val Thr Lys Glu Ser Glu Trp Thr Gly Lys Asn Phe Val Pro

305                 310                 315                 320

Pro Leu Lys Thr Leu Tyr Thr Val Gln Asp Thr Tyr Asn Tyr Asn Asp

                325                 330                 335

Pro Thr Cys Gly Asp Pro Thr Tyr Ile Cys Trp Pro Thr Val Thr Pro

            340                 345                 350

Ser Ser Ala Tyr Val Tyr Lys Gly Gly Lys Lys Ala Ile Ser Gly Trp

        355                 360                 365

Glu Asn Thr Leu Leu Val Pro Ser Leu Lys Arg Gly Val Ile Phe Arg

    370                 375                 380

Ile Lys Leu Asp Pro Thr Tyr Ser Ala Thr Tyr Asp Asp Ala Val Pro
385                 390                 395                 400

Met Phe Lys Ser Asn Asn Arg Tyr Arg Asp Val Ile Ala Ser Pro Asp
                405                 410                 415

Gly Asn Val Leu Tyr Val Leu Thr Asp Thr Ser Gly Asn Val Gln Lys
            420                 425                 430

Asp Asp Gly Ser Val Thr Asn Thr Leu Glu Asn Pro Gly Ser Leu Ile
        435                 440                 445

Lys Phe Thr Tyr Lys Ala Lys
    450                 455

<210>  31
<211>  455
<212>  PRT

<213>  Acinetobacter calcoaceticus

<220>
<221>  MUTAGEN
<222>  (295)..(295)
<223>

<220>
<221>  MUTAGEN
<222>  (342)..(342)
<223>

<220>
<221>  MUTAGEN
<222>  (351)..(351)
<223>

<400>  31

Asp Val Pro Leu Thr Pro Ser Gln Phe Ala Lys Ala Lys Thr Glu Ser
1               5                   10                  15

Phe Asp Lys Lys Val Leu Leu Ser Asn Leu Asn Lys Pro His Ala Leu
            20                  25                  30

Leu Trp Gly Pro Asp Asn Gln Ile Trp Leu Thr Glu Arg Ala Thr Gly
        35                  40                  45
Lys Ile Leu Arg Val Asn Pro Glu Ser Gly Ser Val Lys Thr Val Phe
    50                  55                  60
Gln Val Pro Glu Ile Val Asn Asp Ala Asp Gly Gln Asn Gly Leu Leu
65                  70                  75                  80
Gly Phe Ala Phe His Pro Asp Phe Lys Asn Asn Pro Tyr Ile Tyr Val
                85                  90                  95
Ser Gly Thr Phe Lys Asn Pro Lys Ser Thr Asp Lys Glu Leu Pro Asn
            100                 105                 110
Gln Thr Ile Ile Arg Arg Tyr Thr Tyr Asn Lys Ala Thr Asp Thr Leu
        115                 120                 125

Glu Lys Pro Val Asp Leu Leu Ala Gly Leu Pro Ser Ser Lys Asp His
    130                 135                 140

Gln Ser Gly Arg Leu Val Ile Gly Pro Asp Gln Lys Ile Tyr Tyr Thr
145                 150                 155                 160

Ile Gly Asp Gln Gly Arg Asn Gln Leu Ala Tyr Leu Phe Leu Pro Asn
                165                 170                 175

Gln Ala Gln His Thr Pro Thr Gln Gln Glu Leu Ser Gly Lys Asp Tyr
            180                 185                 190

His Thr Tyr Met Gly Lys Val Leu Arg Leu Asn Leu Asp Gly Ser Ile
        195                 200                 205

Pro Lys Asp Asn Pro Ser Phe Asn Gly Val Ile Ser His Ile Tyr Thr
    210                 215                 220

Leu Gly His Arg Asn Pro Gln Gly Leu Ala Phe Thr Pro Asn Gly Lys
225                 230                 235                 240
Leu Leu Gln Ser Glu Gln Gly Pro Asn Ser Asp Asp Glu Ile Asn Leu
                245                 250                 255
Ile Val Lys Gly Gly Asn Tyr Gly Trp Pro Asn Val Ala Gly Tyr Lys
            260                 265                 270
Asp Asp Ser Gly Tyr Ala Tyr Ala Asn Tyr Ser Ala Ala Ser Asn Lys
        275                 280                 285
Ala Gln Ile Lys Asp Leu Phe Gln Asn Gly Leu Lys Val Ala Ala Gly
    290                 295                 300
Val Pro Val Thr Lys Glu Ser Glu Trp Thr Gly Lys Asn Phe Val Pro
305                 310                 315                 320
Pro Leu Lys Thr Leu Tyr Thr Val Gln Asp Thr Tyr Asn Tyr Asn Asp

                325                 330                 335

Pro Thr Cys Gly Asp Pro Thr Tyr Ile Cys Trp Pro Thr Val Thr Pro

            340                 345                 350

Ser Ser Ala Tyr Val Tyr Lys Gly Gly Lys Lys Ala Ile Ser Gly Trp

        355                 360                 365

Glu Asn Thr Leu Leu Val Pro Ser Leu Lys Arg Gly Val Ile Phe Arg

    370                 375                 380

Ile Lys Leu Asp Pro Thr Tyr Ser Ala Thr Tyr Asp Asp Ala Val Pro

385                 390                 395                 400

Met Phe Lys Ser Asn Asn Arg Tyr Arg Asp Val Ile Ala Ser Pro Asp

                405                 410                 415

Gly Asn Val Leu Tyr Val Leu Thr Asp Thr Ser Gly Asn Val Gln Lys

            420                 425                 430

Asp Asp Gly Ser Val Thr Asn Thr Leu Glu Asn Pro Gly Ser Leu Ile

        435                 440                 445

Lys Phe Thr Tyr Lys Ala Lys

    450                 455

<210> 32

<211> 455

<212> PRT

<213> Acinetobacter calcoaceticus

<220>

<221> MUTAGEN

<222> (169)..(169)

<223>

<220>

<221> MUTAGEN

<222> (295)..(295)

<223>

<220>

<221> MUTAGEN

<222> (342)..(342)

<223>

<400> 32

Asp Val Pro Leu Thr Pro Ser Gln Phe Ala Lys Ala Lys Thr Glu Ser
1               5                   10                  15

Phe Asp Lys Lys Val Leu Leu Ser Asn Leu Asn Lys Pro His Ala Leu
            20                  25                  30

Leu Trp Gly Pro Asp Asn Gln Ile Trp Leu Thr Glu Arg Ala Thr Gly
        35                  40                  45

Lys Ile Leu Arg Val Asn Pro Glu Ser Gly Ser Val Lys Thr Val Phe
    50                  55                  60

Gln Val Pro Glu Ile Val Asn Asp Ala Asp Gly Gln Asn Gly Leu Leu
65                  70                  75                  80

Gly Phe Ala Phe His Pro Asp Phe Lys Asn Asn Pro Tyr Ile Tyr Val
                85                  90                  95
Ser Gly Thr Phe Lys Asn Pro Lys Ser Thr Asp Lys Glu Leu Pro Asn
            100                 105                 110
Gln Thr Ile Ile Arg Arg Tyr Thr Tyr Asn Lys Ala Thr Asp Thr Leu
        115                 120                 125
Glu Lys Pro Val Asp Leu Leu Ala Gly Leu Pro Ser Ser Lys Asp His
    130                 135                 140
Gln Ser Gly Arg Leu Val Ile Gly Pro Asp Gln Lys Ile Tyr Tyr Thr
145                 150                 155                 160
Ile Gly Asp Gln Gly Arg Asn Gln Phe Ala Tyr Leu Phe Leu Pro Asn
                165                 170                 175

Gln Ala Gln His Thr Pro Thr Gln Gln Glu Leu Ser Gly Lys Asp Tyr
            180                 185                 190
His Thr Tyr Met Gly Lys Val Leu Arg Leu Asn Leu Asp Gly Ser Ile
        195                 200                 205
Pro Lys Asp Asn Pro Ser Phe Asn Gly Val Ile Ser His Ile Tyr Thr
    210                 215                 220
Leu Gly His Arg Asn Pro Gln Gly Leu Ala Phe Thr Pro Asn Gly Lys
225                 230                 235                 240
Leu Leu Gln Ser Glu Gln Gly Pro Asn Ser Asp Asp Glu Ile Asn Leu
                245                 250                 255
Ile Val Lys Gly Gly Asn Tyr Gly Trp Pro Asn Val Ala Gly Tyr Lys
            260                 265                 270

Asp Asp Ser Gly Tyr Ala Tyr Ala Asn Tyr Ser Ala Ala Ser Asn Lys
        275                 280                 285

Ala Gln Ile Lys Asp Leu Glu Gln Asn Gly Leu Lys Val Ala Ala Gly
    290                 295                 300

Val Pro Val Thr Lys Glu Ser Glu Trp Thr Gly Lys Asn Phe Val Pro
305                 310                 315                 320

Pro Leu Lys Thr Leu Tyr Thr Val Gln Asp Thr Tyr Asn Tyr Asn Asp
                325                 330                 335

Pro Thr Cys Gly Asp Pro Thr Tyr Ile Cys Trp Pro Thr Val Ala Pro
            340                 345                 350

Ser Ser Ala Tyr Val Tyr Lys Gly Gly Lys Lys Ala Ile Ser Gly Trp
        355                 360                 365

Glu Asn Thr Leu Leu Val Pro Ser Leu Lys Arg Gly Val Ile Phe Arg

    370                 375                 380

Ile Lys Leu Asp Pro Thr Tyr Ser Ala Thr Tyr Asp Asp Ala Val Pro
385                 390                 395                 400

Met Phe Lys Ser Asn Asn Arg Tyr Arg Asp Val Ile Ala Ser Pro Asp
                405                 410                 415

Gly Asn Val Leu Tyr Val Leu Thr Asp Thr Ser Gly Asn Val Gln Lys
            420                 425                 430

Asp Asp Gly Ser Val Thr Asn Thr Leu Glu Asn Pro Gly Ser Leu Ile
        435                 440                 445

Lys Phe Thr Tyr Lys Ala Lys
    450                 455

<210> 33
<211> 455

<212>  PRT
<213>  Acinetobacter calcoaceticus

<220>
<221>  MUTAGEN
<222>  (75)..(75)
<223>

<220>
<221>  MUTAGEN
<222>  (295)..(295)
<223>

<220>
<221>  MUTAGEN
<222>  (342)..(342)
<223>

<220>
<221>  MUTAGEN
<222>  (351)..(351)
<223>

<400>  33

Asp Val Pro Leu Thr Pro Ser Gln Phe Ala Lys Ala Lys Thr Glu Ser
1               5                   10                  15

Phe Asp Lys Lys Val Leu Leu Ser Asn Leu Asn Lys Pro His Ala Leu
            20                  25                  30

Leu Trp Gly Pro Asp Asn Gln Ile Trp Leu Thr Glu Arg Ala Thr Gly
        35                  40                  45

Lys Ile Leu Arg Val Asn Pro Glu Ser Gly Ser Val Lys Thr Val Phe
    50                  55                  60

Gln Val Pro Glu Ile Val Asn Asp Ala Asp Trp Gln Asn Gly Leu Leu
65                  70                  75                  80

Gly Phe Ala Phe His Pro Asp Phe Lys Asn Asn Pro Tyr Ile Tyr Val
                85                  90                  95

Ser Gly Thr Phe Lys Asn Pro Lys Ser Thr Asp Lys Glu Leu Pro Asn

            100                 105                 110

Gln Thr Ile Ile Arg Arg Tyr Thr Tyr Asn Lys Ala Thr Asp Thr Leu
        115                 120                 125

Glu Lys Pro Val Asp Leu Leu Ala Gly Leu Pro Ser Ser Lys Asp His
    130                 135                 140

Gln Ser Gly Arg Leu Val Ile Gly Pro Asp Gln Lys Ile Tyr Tyr Thr
145                 150                 155                 160

Ile Gly Asp Gln Gly Arg Asn Gln Leu Ala Tyr Leu Phe Leu Pro Asn
                165                 170                 175

Gln Ala Gln His Thr Pro Thr Gln Gln Glu Leu Ser Gly Lys Asp Tyr
            180                 185                 190

His Thr Tyr Met Gly Lys Val Leu Arg Leu Asn Leu Asp Gly Ser Ile
        195                 200                 205

Pro Lys Asp Asn Pro Ser Phe Asn Gly Val Ile Ser His Ile Tyr Thr
    210                 215                 220
Leu Gly His Arg Asn Pro Gln Gly Leu Ala Phe Thr Pro Asn Gly Lys
225                 230                 235                 240
Leu Leu Gln Ser Glu Gln Gly Pro Asn Ser Asp Asp Glu Ile Asn Leu
                245                 250                 255
Ile Val Lys Gly Gly Asn Tyr Gly Trp Pro Asn Val Ala Gly Tyr Lys
            260                 265                 270
Asp Asp Ser Gly Tyr Ala Tyr Ala Asn Tyr Ser Ala Ala Ser Asn Lys
        275                 280                 285
Ala Gln Ile Lys Asp Leu Glu Gln Asn Gly Leu Lys Val Ala Ala Gly
    290                 295                 300

Val Pro Val Thr Lys Glu Ser Glu Trp Thr Gly Lys Asn Phe Val Pro
305                 310                 315                 320

Pro Leu Lys Thr Leu Tyr Thr Val Gln Asp Thr Tyr Asn Tyr Asn Asp
                325                 330                 335

Pro Thr Cys Gly Asp Pro Thr Tyr Ile Cys Trp Pro Thr Val Thr Pro
            340                 345                 350

Ser Ser Ala Tyr Val Tyr Lys Gly Gly Lys Lys Ala Ile Ser Gly Trp
        355                 360                 365

Glu Asn Thr Leu Leu Val Pro Ser Leu Lys Arg Gly Val Ile Phe Arg
    370                 375                 380

Ile Lys Leu Asp Pro Thr Tyr Ser Ala Thr Tyr Asp Asp Ala Val Pro
385                 390                 395                 400

Met Phe Lys Ser Asn Asn Arg Tyr Arg Asp Val Ile Ala Ser Pro Asp
                405                 410                 415

Gly Asn Val Leu Tyr Val Leu Thr Asp Thr Ser Gly Asn Val Gln Lys
            420                 425                 430

Asp Asp Gly Ser Val Thr Asn Thr Leu Glu Asn Pro Gly Ser Leu Ile
        435                 440                 445

Lys Phe Thr Tyr Lys Ala Lys
    450                 455

<210> 34
<211> 455
<212> PRT
<213> Acinetobacter calcoaceticus

<220>
<221> MUTAGEN
<222> (342)..(342)
<223>

<220>

<221>  MUTAGEN

<222>  (351)..(351)

<223>

<400>  34

Asp Val Pro Leu Thr Pro Ser Gln Phe Ala Lys Ala Lys Thr Glu Ser
1               5                   10                  15

Phe Asp Lys Lys Val Leu Leu Ser Asn Leu Asn Lys Pro His Ala Leu
            20                  25                  30

Leu Trp Gly Pro Asp Asn Gln Ile Trp Leu Thr Glu Arg Ala Thr Gly
        35                  40                  45

Lys Ile Leu Arg Val Asn Pro Glu Ser Gly Ser Val Lys Thr Val Phe
    50                  55                  60

Gln Val Pro Glu Ile Val Asn Asp Ala Asp Gly Gln Asn Gly Leu Leu

65                  70                  75                  80

Gly Phe Ala Phe His Pro Asp Phe Lys Asn Asn Pro Tyr Ile Tyr Val
                85                  90                  95

Ser Gly Thr Phe Lys Asn Pro Lys Ser Thr Asp Lys Glu Leu Pro Asn
            100                 105                 110

Gln Thr Ile Ile Arg Arg Tyr Thr Tyr Asn Lys Ala Thr Asp Thr Leu
        115                 120                 125

Glu Lys Pro Val Asp Leu Leu Ala Gly Leu Pro Ser Ser Lys Asp His
    130                 135                 140

Gln Ser Gly Arg Leu Val Ile Gly Pro Asp Gln Lys Ile Tyr Tyr Thr
145                 150                 155                 160

Ile Gly Asp Gln Gly Arg Asn Gln Leu Ala Tyr Leu Phe Leu Pro Asn
                165                 170                 175

Gln Ala Gln His Thr Pro Thr Gln Gln Glu Leu Ser Gly Lys Asp Tyr
            180                 185                 190
His Thr Tyr Met Gly Lys Val Leu Arg Leu Asn Leu Asp Gly Ser Ile
        195                 200                 205
Pro Lys Asp Asn Pro Ser Phe Asn Gly Val Ile Ser His Ile Tyr Thr
    210                 215                 220
Leu Gly His Arg Asn Pro Gln Gly Leu Ala Phe Thr Pro Asn Gly Lys
225                 230                 235                 240
Leu Leu Gln Ser Glu Gln Gly Pro Asn Ser Asp Asp Glu Ile Asn Leu
                245                 250                 255
Ile Val Lys Gly Gly Asn Tyr Gly Trp Pro Asn Val Ala Gly Tyr Lys
            260                 265                 270

Asp Asp Ser Gly Tyr Ala Tyr Ala Asn Tyr Ser Ala Ala Ser Asn Lys
        275                 280                 285

Ala Gln Ile Lys Asp Leu Gly Gln Asn Gly Leu Lys Val Ala Ala Gly
    290                 295                 300

Val Pro Val Thr Lys Glu Ser Glu Trp Thr Gly Lys Asn Phe Val Pro
305                 310                 315                 320

Pro Leu Lys Thr Leu Tyr Thr Val Gln Asp Thr Tyr Asn Tyr Asn Asp
                325                 330                 335

Pro Thr Cys Gly Asp Pro Thr Tyr Ile Cys Trp Pro Thr Val Thr Pro
            340                 345                 350

Ser Ser Ala Tyr Val Tyr Lys Gly Gly Lys Lys Ala Ile Ser Gly Trp
        355                 360                 365

Glu Asn Thr Leu Leu Val Pro Ser Leu Lys Arg Gly Val Ile Phe Arg
    370                 375                 380

Ile Lys Leu Asp Pro Thr Tyr Ser Ala Thr Tyr Asp Asp Ala Val Pro
385                 390                 395                 400

Met Phe Lys Ser Asn Asn Arg Tyr Arg Asp Val Ile Ala Ser Pro Asp
                405                 410                 415

Gly Asn Val Leu Tyr Val Leu Thr Asp Thr Ser Gly Asn Val Gln Lys
            420                 425                 430

Asp Asp Gly Ser Val Thr Asn Thr Leu Glu Asn Pro Gly Ser Leu Ile
        435                 440                 445

Lys Phe Thr Tyr Lys Ala Lys
    450                 455